# EUROPÄISCHE PATENTSCHRIFT

(11) **EP 1 362 023 B1**
(45) Veröffentlichungstag und Bekanntmachung des Hinweises auf die Patenterteilung: **15.04.2009**
(21) Anmeldenummer: 02719851.4
(22) Anmeldetag: 15.02.2002
(51) Int. Cl.: C07C 45/51, C07C 49/307, C07C 49/547, C07C 35/205, C07C 29/40, C07C 29/42

(54) **VERFAHREN ZUR HERSTELLUNG MAKROCYCLISCHER KETONE**
METHOD FOR PRODUCING MACROCYCLIC KETONES
PROCEDE DE PRODUCTION DE CETONES MACROCYCLIQUES

(30) Priorität: 22.02.2001 EP 01103613
(43) Veröffentlichungstag der Anmeldung: 19.11.2003
(73) Patentinhaber: Givaudan SA, 1214 Vernier (CH)
(72) Erfinder: FRATER, Georg, CH-8400 Winterthur (CH); NAGEL, Matthias, CH-8600 Duebendorf (CH)
(74) Vertreter: Givaudan Patents
(86) Internationale Anmeldenummer: PCT/EP2002/001644
(87) Internationale Veröffentlichungsnummer: WO 2002/068372

(56) Entgegenhaltungen:
- EP-A- 0 955 285
- THIES R W & WILLS M T: "Oxy-Cope Rearrangements of Medium-Sized Rings" TETRAHEDRON LETTERS, Nr. 7, 1970, Seiten 513-516, XP000996741 in der Anmeldung erwähnt
- THIES R W & BILLIGMEIER J E: "Thermal Rearrangements of cis- and trans-Trimethylsiloxy-1-vinylcyclododec-3- ene. Ring Strain Effects for the Siloxy-Cope Rearrangement" JOURNAL OF THE AMERICAN CHEMICAL SOCIETY, Bd. 96, Nr. 4, 1974, Seiten 200-203, XP000999025 in der Anmeldung erwähnt
- DATABASE CROSSFIRE BEILSTEIN [Online] Beilstein; Beilstein Registry Number 2440894, 5. Juli 1989 (1989-07-05) XP002168440 & KHIM. GETEROTSIKL. SOEDIN., Bd. 8, 1972, Seite 170

## Beschreibung

Die vorliegende Erfindung betrifft ein neues Thermo-Isomerisierungsverfahren zur schnellen und einfachen Herstellung von makrocyclischen Ketonen.

Makrocyclischen Ketonen kommen in der Parfümerie aufgrund ihrer moschusartigen Geruchseigenschaften eine erhebliche Wichtigkeit zu. Kommerzielle Bedeutung als Moschusketone haben dabei bisher insbesondere Cyclopentadecanon (Exalton^{®}) , 3-Methylcyclopentadecanon (Muskon), Cycloheptadec-9-en-1-on (Zibeton) und (*E*/*Z*)-Cyclohexadec-5-en-1-on (Ambretone^{®} , Musk TM II^{®}). Wie aus der Fachliteratur bekannt ist, können solche makrocyclische Ketone durch vielstufige Synthesen hergestellt werden, die im wesentlichen auf zwei Grundmethoden beruhen:
a) Makrocyclisierungsreaktionen (wie beispielsweise die sogenannte Acyloin-Kondensation von α,ω-Diestern mit anschliessender Reduktion oder die intramolekulare Olefinmetathese (Ringschluss-Metathese, RCM) etc.). Da Polymerisationsprozesse vermieden werden sollen, werden die meisten Makrocyclisierungsreaktionen nur unter der Anwendung hoher Verdünnungen durchgeführt und sind daher für Umsetzungen im Grossmassstab zu kostenintensiv.
b) Ringerweiterungsreaktionen, die entweder auf einer mehrstufigen Sequenz von Anellierungs- und Fragmentierungs-Reaktionen beruhen, oder aber auf sigmatropen [3.3]-Umlagerungsreaktionen (z.B. Oxy-*Cope*-Reaktion von 1,2-Divinylcycloalkan-1-olen, wie zum Beispiel in EP 0 955 285 beschrieben), bzw. auch [1.3]-Verschiebungsreaktionen bei 1-Vinylcycloalk-3-en-1-olen basieren (W. Thies, P. Daruwala, J. Org. Chem. 1987, 52, 3798 - 3806).

Ringerweiterungsreaktionen um 2 Kohlenstoffatome nach dem Prinzip einer [1.3]-Verschiebungsreaktion werden in mehreren wissenschaftlichen Artikeln beschrieben (beispielsweise in Tetrahedron Lett. 1970, 513 - 516). Bei 1-Vinyl-cyloalk-3-en-1-olen mit neun- bis dreizehngliedrigem Ringsystem und endocyclischer, homoallylischer Doppelbindung in Position 3, wurden unter den thermischen Bedingungen für Oxy-*Cope-*Umlagerungen, anstelle von [3.3]-Umlagerungsprodukten (Ringerweiterung um vier C-Atome) Ringerweiterungen bevorzugt im Sinne einer [1.3]-Verschiebung (Ringerweiterung um zwei Kohlenstoffatome) beobachtet. Die Ausbeute beträgt maximal 25%, da als unerwünschte Reaktion die Eliminierung von Wasser eintritt. Signifikant bessere Ausbeuten (50-80%) werden beschrieben, wenn der Vinylalkohol vor dem Erhitzen in den entsprechenden Trimethylsilylether übergeführt wurde. Dieselben Produkte wurden erhalten, wenn die Umlagerungsreaktion mit dem entsprechenden doppelt ungesättigten Kaliumalkoholat unter stark anionischen Bedingungen (2 Mol-Equiv. Kaliumhydrid in HMPA bei 25 - 100°C) ausgeführt wurde.

In J. Am. Chem. Soc. 1974, 964, 200 - 203 wird ausdrücklich darauf hingewiesen, dass sowohl beim Trimethylsilylether-Derivat als besonders auch beim nicht-derivatisierten Vinylalkohol selbst eine analoge [1.3]-Verschiebungsreaktion bei Temperaturen bis 320°C nicht mehr beobachtet werden konnte, sobald die homoallylische endocyclische Doppelbindung (in Position 3) entfernt wurde. Die Anwendung zusätzlich forcierter Reaktionsbedingungen (Reaktionstemperaturen bis 420°C) führte in beiden Fällen hauptsächlich zur Bildung von Alkenen. (Eliminierung von Wasser). In J. Org. Chem. 1978, 43, 1050-1057, wird beschrieben, dass ebenso bei der Anwendung anionischer Umlagerungsbedingungen, also durch Behandlung des im Ringsystem gesättigten Vinylalkohols mit Kaliumhydrid in HMPA für 4 bzw. 24 h bei Raumtemperatur, keine Produkte einer Ringerweiterung beobachtet werden konnten.

Die zu den Vinylcyclopropanen strukturverwandten 1-Vinylcyclopropanole lagern bereits beim Erhitzen auf nur 100°C innerhalb kurzer Zeit unter einer [1.2]-Wanderung irreversibel in 2-substituierte Cyclobutanone um. Werden dagegen jedoch anstelle der Vinylcyclopropanole deren Trimethylsilylether-Derivate (1-Trimethylsilyloxy-1-vinylcyclopropane) erhitzt, so können diese wiederum unter einer [1.3]-Wanderung in Cyclopentanone übergeführt werden (J. Am. Chem. Soc. 1973, 95, 5311 - 5321; und J. Org. Chem. 1981, 46, 506 - 509).

In J. Org. Chem, 1978, 43, 4903-4905 sowie in J. Org. Chem, 1980, 45, 2460 - 2468 werden Ringerweiterung um zwei Kohlenstoffatome auch im Fall speziell funktionalisierter makrocyclischer Dithiaspiroketon-Derivate beschrieben (z.B. 1.5-Dithiaspiro[5.12]octadecan-7-on). Diese beschriebene Methode lässt sich nicht mehr auf kleinere Ringsysteme anwenden, wie beispielsweise auf Cyclododecanon als Ausgangsmaterial.

Aufgabe der vorliegenden Erfindung ist ein einfaches und kostengünstiges Verfahren zur Herstellung von makrocyclischen Ketonen.

Überraschenderweise wurde festgestellt, dass durch das erfindungsgemässe Therme-Isomerisierungsverfahren, makrocyclische Ketone der Formel Ia oder Ib in der Gasphase bei Temperaturen oberhalb von 500°C schnell und in einfacher Weise direkt mit einer hohen Ausbeute hergestellt werden können. In den makrocyclischen Ketone der Formel Ia und Ib 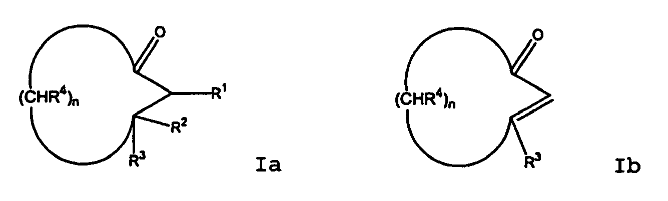 stehen
R¹ R², R³ unabhängig voneinander für Wasserstoff oder eine C₁ bis C₆-Alkyl-Gruppe,
R⁴ für Wasserstoff, eine lineare oder verzweigte C₁ bis C₄-AlkylGruppe,
wobei n eine ganze Zahl ist, und
n 7 bis 14 ist, und
und in Formel Ia können R¹ und R² oder R² und R³ unabbhängig voneinander einen Ring bilden.

Die um zwei Kohlenstoffatome ringerweiterten Ketone der Formel Ia oder Ib werden durch Überführung eines makrocyclischen tertiären Allyl- bzw. Propargylalkohols der Formel IIa oder IIb 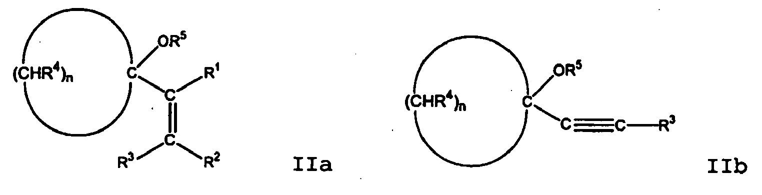
wobei R¹, R², R³, R⁴, und n die gleiche Bedeutung haben wie oben und R⁵ entweder Wasserstoff oder Trialkylsilyl oder ein Alkalimetallkation darstellt,
unter reduziertem Druck bei 100-300°C in die Gasphase, und
durch Erhitzen des in die Gasphase überführen makrocyclischen tertiären Allyl- bzw. Propargylalkohols der Formel IIa oder IIb auf Temperaturen von 500 bis 700°C, und
Hydrolyse des Trialkylsilylethers in das entsprechende Keton der Formel Ia oder Ib, falls R⁵ ein Trialkylsilyl, hergestellt.

Die Grösse des Ringsystems wird mithin durch n beschrieben. Falls n=7 werden aus 8-gliedrigen cyclischen Alkoholen 10-gliedrige cyclische Ketone. Im Falle von n=14 können entsprechend aus 15-gliedrigen cyclischen Alkoholen 17-gliedrige cyclische Ketone erhalten werden.

C₁-C₆-Alkyl ist beispielsweise Methyl, Ethyl, Propyl, Isopropyl, Butyl, sec.- oder tert.-Butyl, Pentyl oder Hexyl, wobei Methyl besonders bevorzugt wird.

Das beschriebene Verfahren ermöglicht, je nach Art der Substituenten an der Vinylgruppe des als Ausgangsmaterial eingesetzten tertiären cyclischen Alkohols der Formel IIa, so sich die Substitutenten von Wasserstoff unterscheiden, gleichzeitig die Herstellung von ringerweiterten und zusätzlich regioselektiv substiuierten makrocyclischen Ketonen. Die Positionen dieser Substituenten in den makrocyclischen Ketonen befinden sich wie in Formel Ia dargestellt an den beiden aufeinanderfolgenden, zum Kohlenstoffatom der Carbonylgruppe (betrachtet als C(1)) benachbarten Kohlenstoffatomen und verteilen sich in folgender Weise: R¹ befindet sich am Kohlenstoffatom direkt benachbart zur Carbonylgruppe (also αständig) an C(2) und sowohl R² als auch R³ befinden sich jeweils auf der gleichen Ringhälfte des makrocyclischen Ketons um zwei Kohlenstoffatome von der Carbonylgruppe entfernt (also βständig) an C(3), direkt benachbart zu C(2). Werden bei dem beschriebenen Verfahren anstelle von tertiären cyclischen Allylalkoholen entsprechende makrocyclische Propargylalkohole der Formel IIb eingesetzt, so werden makrocyclische Ketone erhalten, die eine Doppelbindung in direkter Konjugation zur Carbonylgruppe enthalten (α,β-ungesättigte Ketone). Befindet sich am Ringsystem des cyclischen Alkohols ein zusätzlicher Substituent R⁴, so sich R⁴ von Wasserstoff unterscheidet, können zudem regioisomere ringerweiterte makrocyclische Ketone erhalten werden, abhängig davon auf welcher Hälfte des cyclischen Systems die beiden Kohlenstoffatome der ursprünglichen Vinylgruppe des Ausgangsmaterials gemäss der zuvor beschriebenen Weise inkorporiert werden. Dies gilt sinngemäss auch dann, wenn R⁴ mehrere Substituenten repräsentiert.

Da mit dem beschriebenen Thermo-Isomerisierungsverfahren aus tertiären cyclischen Allyl- bzw. Propargylalkoholen makrocyclische Ketone hergestellt werden können, die sich ihrerseits wiederum effizient und in einfacher Weise nach den bekannten Methoden erneut in cyclische tertiäre Allyl- bzw. Propargylalkohole überführen lassen, die dann ihrerseits wiederum erneut als Ausgangsmaterial im beschriebenen Thermo-Isomerisierungsverfahren eingesetzt werden können, bietet das beschriebene Verfahren durch repetitive Anwendung zusätzlich die Möglichkeit, durch Iteration jeweils auch die um vier, sechs, acht usw. Kohlenstoffatome ringerweiterten makrocyclischen Ketone herzustellen, wobei pro repetitivem Zyklus lediglich zwei Synthesestufen erforderlich sind.

Das beschriebenen Verfahren lässt sich in gleicher Weise auch auf die analogen veretherten Derivate der zugrundeliegenden makrocyclischen tertiären Alkohole anwenden, beispielsweise auf Trialkylsilylether, insbesondere auf Trimethylsilylether (wenn also R⁵ in Formel IIa oder IIb eine Trimethylsilylgruppe repräsentiert), wobei dann in analoger Weise zunächst jedoch jeweils Gemische der entsprechenden ringerweiterten cyclischen Trimethylsilylenolether erhalten werden. Die Hydrolyse dieser Trimethylsilylenolethergemische führt dann ebenfalls wieder zu den gleichen Ketonen, die aus den entsprechenden zugrundeliegenden Alkoholen in direkter Weise, also ohne vorherige Derivatisierung durch eine geeignete Silylgruppe sowie ohne die damit zusätzlich erforderliche Hydrolysestufe erhalten werden können.

Durch die Ausführung der Thermo-Isomerisierung in der Gasphase kann das erfindungsgemässe Verfahren ohne Verwendung von Lösungsmitteln und damit dem Umweltschutz gerecht werdend erfolgen, sofern das Ausgangsmaterial direkt in die Gasphase verdampft und in die Reaktor-Einheit übergeführt wird. Eine zweckmässige Auslegung der Apparatur erlaubt es zudem, das beschriebene Thermo-Isomerisierungsverfahren auch kontinuierlich führen zu können und damit potentiell auch eine Prozessautomation zu ermöglichen. In der wissenschaftlichen Fachliteratur finden sich dazu vielfache Beschreibungen über unterschiedliche Apparaturen zur Durchführung von verschiedenartigsten analogen chemischen Umwandlungsprozessen in der Gasphase unter Anwendung von Temperaturen von bis zu ca. 1000°C (Gasphasen-Strömungsthermolyse-, bzw. Kurzzeit-Vakuum-Pyrolyse-Apparaturen).

Das erfindungsgemässe Verfahren zur Herstellung von makrocyclischen Ketonen beruht auf der Thermo-Isomerisierung von tertiären makrocyclischen Allylalkoholen oder Propargylalkoholen in der Gasphase bei Temperaturen von 500 bis 700°C. Zur Durchführung des erfindungsgemässen Verfahrens wird der als Ausgangsmaterial eingesetzte Alkohol in einer Verdampfungseinheit entweder vorgelegt oder aber über eine Dosiereinrichtung, wie beispielsweise eine Dosier- oder Spritzenpumpe aus einem Vorratsgefäss bevorzugt entweder unverdünnt oder auch gelöst in einem geeigneten inerten Lösungsmittel (wie beispielsweise Xylen) kontinuierlich zugegeben und unter reduziertem Druck in Abhängigkeit vom Siedepunkt des eingesetzten Ausgangsmaterials auf Temperaturen im Bereich von ca. 100 - 300°C, bevorzugt im Bereich von 120 - 250°C erhitzt und dabei in die Gasphase übergeführt. Der in der Verdampfungseinheit vorerhitzte Alkohol wird nun in der Gasphase, gegebenenfalls auch unter Anwendung eines regulierbaren Inertgasstromes, wobei das Inertgas beispielsweise Stickstoff, Argon oder Helium sein kann, und bei vermindertem Druck durch eine geeignet dimensionierte und zweckmässig geformte Reaktor-Einheit geleitet, die auf Temperaturen zwischen 500 und 700°C geheizt ist, wobei der eingesetzte makrocyclische tertiäre Allyl- bzw. Propargylalkohol gemäss der vorliegenden Beschreibung des erfindungsmässigen Thermo-Isomerisierungsprozesses in das entsprechende makrocyclische Keton umgewandelt wird.

Die Reaktor-Einheit hat zweckmässigerweise im allgemeinen röhrenartige Form, besteht vorteilhafterweise aus einem thermostabilen und den Ablauf der Isomerisierungsreaktion nicht störenden inerten Material, wie beispielsweise bestimmten hochschmelzenden Glassorten, und kann horizontal oder vertikal oder auch beliebig geneigt angeordnet sein, und unabhängig von der Verdampfungseinheit auf allgemein bekannte Weise, beispielsweise mit einem elektrischen Heizmantel beheizt werden.

Der für das beschriebene Thermo-Isomerisierungsverfahren erforderliche Temperaturbereich hängt dabei gleichzeitig von mehreren Faktoren wie beispielsweise dem herrschenden Druck im Reaktor, der Gestalt und den Abmessungen des Reaktor-Gefässes, der Grösse des Inertgasstroms (Flow) sowie der Zugabegeschwindigkeit bzw. Verdampfungsrate des Ausgangsmaterials bzw. von dessen Lösungen sowie vom Lösungsmittel ab und liegt bevorzugt im Bereich von 500 bis 700°C. Unterhalb von ca. 450°C wird der Thermo-Isomerisierungsprozess so langsam, dass hauptsächlich unverändertes Ausgangsmaterial und gegebenenfalls Dehydratisierungsprodukte gefunden werden, während bei Temperaturen oberhalb 700°C in zunehmendem Mass Zersetzungsprodukte und störende Nebenreaktionen beobachtet werden. Der bevorzugte Temperaturbereich für eine möglichst vollständige Umwandlung der im beschriebenen Thermo-Isomerisierungsverfahren eingesetzten Ausgangsmaterialien liegt in direkter Abhängigkeit von den genannten individuellen Reaktorparametern häufig oberhalb von 550°C, besonders optimal im Bereich von 570 - 670°C, insbesondere wenn ohne Lösungsmittel mit einem schwachen Inertgasstrom oder im Hochvakuum ohne Inertgasstrom und ohne eine Füllkörperpackung gearbeitet wird. Vorzugsweise wird das beschriebene Thermo-Isomerisierungverfahren bei vermindertem Druck durchgeführt, besonders vorteilhaft bei einem Vakuum im Bereich von ca. 1 bis 10 mbar (1 - 10 hPa), jedenfalls zweckmässigerweise unterhalb des Sättigungsdampfdruckes des als Ausgangsmaterial eingesetzten Alkohols, jedoch lässt sich im Inertgasstrom auch noch bei weniger stark vermindertem Druck, wie beispielsweise am Wasserstrahlvakuum bzw. mit Labormembranvakuumpumpen die gewünschte Produktbildung beobachten. Der Druck in der Apparatur und damit gleichzeitig die Kontaktzeit in der Reaktor-Einheit kann zusätzlich durch die Regulierung des Inertgasstromes, beziehungsweise bei Injektion von flüssigen Ausgangsmaterialien durch die Zugabegeschwindigkeit oder bei zunächst festen Ausgangsmaterialien durch die Verdampfungsrate beeinflusst werden.

In der nachgeschalteten Kondensator-Einheit werden die durch das Thermo-Isomerisierungsverfahren erhaltenen gasförmigen Reaktionsprodukte mittels eines geeigneten Mediums nach bekannten Methoden auf Raumtemperatur oder darunter abgekühlt und dabei wieder verflüssigt (bzw. in einzelnen Fällen resublimiert) und schliesslich in einem Auffangbehältnis gesammelt. Eine Auslassventil am Auffangbehältnis und ein weiteres Ventil (Hahn) am vom Auffangbehältnis abnehmbaren Sammelbehältnis erlaubt nach dem Prinzip des *Normag-Thiele-*Vorstosses das Sammeln und die Entnahme zumindest der flüssigen Reaktionsprodukte auch unter weiterbestehendem vermindertem Druck innerhalb der beschriebenen Thermo-Isomerisierungsapparatur, insbesondere in der Verdampfer-, Reaktor- und Kondensator-Einheit, so dass auch ein kontinuierlicher Dauerbetrieb der Apparatur gewährleistet werden kann. Der verminderte Druck in der Apparatur wird durch eine Vakuumpumpen-Einheit mit geeigneter Saugkapazität, mit besonderem Vorteil mittels einer Hochvakuumpumpe erzeugt, wobei zwischen Vakuumpumpe und Apparatur zum Auffangen leichtflüchtiger Nebenproduktkomponenten noch weitere Kühlfallen mit einem geeigneten Kühlmedium geschaltet sein können.

Durch das erfindungsgemässe Verfahren konnten folgende Produkte neu hergestellt werden:
2-Methylcyclotetradecanon
3-Methylcycloheptadecanon
5-Methylcycloheptadecanon
3-Methylcycloheptadecanon
4-Ethylcyclotetradecanon
3,4-Dimethylcyclotetradecanon.

Die als Ausgangsmaterialien für den Thermo-Isomerisierungsprozess erforderlichen tertiären makrocyclischen Allylalkohole der Formel IIa lassen sich leicht nach bekannten Methoden, beispielsweise bevorzugt durch Addition von geeigneten Organometall-Alkenylverbindungen wie Magnesium- oder Lithium-1-alkenylen an die entsprechenden makrocyclischen Ketone herstellen. Die Ausbeuten an makrocyclischen Allyl- bzw. Propargylalkoholen für die Addition von gängigen Vinyl-*Grignard-*Reagenzien, wie beispielsweise Vinylmagnesiumchlorid bzw. Vinylmagnesiumbromid, bzw. z. B. auch von entsprechenden 1- bzw. 2-substituierten Vinyl-*Grignard*-Verbindungen bzw. Metall-Alkinid-Derivaten, wie sie als Substituenten an C(1) der tertiären makrocyclischen Alkohole der Formel IIa dargestellt sind, und wobei R¹ bis R³ die in der Beschreibung angegebene Bedeutung haben, liegen für makrocyclische Ketone - sofern die entsprechenden Verbindungen überhaupt bereits beschrieben und in der zugänglichen Literatur überhaupt Angaben zu den erhaltenen Ausbeuten niedergelegt sind - im Bereich bis maximal ca. 70%.

Bessere Ausbeuten an makrocyclischen Allylalkohole der Formel IIa, meist im Bereich um oder auch über ca. 90% kann unter Übertragung einer Vorkomplexierungsmethode auf makrocyclische Ketone erhalten werden. Dies kann mit katalytischen bzw. substöchiometrischen oder auch stöchiometrischen Mengen einer wasserfreien Lewissäure, wiezum Beispiel. des Certrichlorids (CeCl₃) bei Temperaturen im Bereich von ca. 0-40°C erreicht werden, wobei verbesserte Ausbeuten an tertiären Allylalkoholen bei der nachfolgenden Addition des eingesetzen Organometall-Alkenyls erhalten wurden. Die Zugabe von 1.01 bis ca. 2 mol-Equivalenten, vorzugsweise von 1.5 bis 1.8 mol-Equivalenten der Organometallalkenyl-Lösung in absolutem THF zur Suspension des in der beschriebenen Weise vorkomplexierten makrocyclischen Ketons verläuft in der Regel mit merklicher Wärmetönung und erfolgt daher vorteilhafterweise so, dass die Geschwindigkeit der Zugabe in Abhängigkeit von der vorhandenen Kühlkapazität des Reaktorsystems so gewählt wird, dass die Temperatur des vorgekühlten Reaktionsgemisches 30 bis ca 40°C nicht überschreitet. Anschliessend wird die Temperatur unter Rührern noch für ca. 10 bis 120 Minuten bei 35 bis 40 °C gehalten, und nach Kontrolle des Reaktionsverlaufs beispielsweise durch Gaschromatographie des Reaktionsgemisches, gegebenenfalls nochmals 0.1 bis 0.2 mol-Equivalente der Alkenyl- bzw. Alkinyl-Verbindung bis zur möglichst vollständigen Umsetzung des eingesetzten Ketons zugegeben. Der nach Hydrolyse des Reaktionsgemisches erhaltene tertiäre makrocyclische Allyl- bzw. Propargylalkohol wird entweder durch Destillation im Hochvakuum bzw. durch Umkristallisation oder Chromatographie gereinigt, oder auch als Rohprodukt direkt für die nachfolgenden Thermo-Isomerisierung als Ausgangsmaterial eingesetzt.

Durch die oben beschriebene Methode konnten folgende Ausgangsmaterialien für den Thermo-Isomerisierungsprozess erforderlichen tertiären makrocyclischen Allylalkohole der Formel IIa oder Propargylalkohole der Formel IIb neu hergestellt werden:
1-Vinyl-1-cycloundecanol
1-Vinyl-1-cyclotridecanol
1-Vinyl-1-cyclotetradecanol
1-Vinyl-1-cyclopentadecanol
(syn/anti)-2-Methyl-1-vinyl-1-cyclododecanol
(syn/anti)-3-Methyl-1-vinyl-1-cyclopentadecanol
(E/Z)-1-(1-Propen-1-yl)cyclododecanol
(E/Z)-1-(1-Propenyl)cyclotridecanol
(E/Z)-1-(1-Propenyl)cyclotetradecanol
(E/Z)-1-(1-Propen-1-yl)cyclopentadecanol
1-(1-Methylethenyl)cycloundecanol
1-(2-Methyl-1-popenyl)cyclododecanol
(E/Z)-1-(2-Buten-2-yl)cyclododecanol
1-Ethinylcyclotridecanol
1-(1-Propinyl)dodecanol
(*E*/*Z*)-1-(Trimehylsilyloxy)-1-(1-propenyl)cyclododecanol).

### Beispiele

### I. Alkenyl- und 1-Alkinyl-Grignard-Reaktionen:

### a) Trocknung von Certrichlorid:

100 g Cer(III)chlorid Heptahydrat (Fluka) (0.268 mol) wurden unter ständigem Rotieren in einem Büchi-Kugelrohrofen am Hochvakuum getrocknet, indem zunächst für 5 - 6 h bei einer Luftbadtemperatur von 70 - 80°C, sodann für 3 - 4 h bei 110 - 120°C und schliesslich über Nacht (ca. 12 h) auf 150 - 160°C erhitzt wurde. Das abgeschiedene Wasser wurde in Kühlfallen (Flüssigstickstoff-Kühlung) gesammelt, wobei diese mehrmals gewechselt wurden, bis schliesslich keine Kondensatbildung mehr beobachtet wurde. Nach dem Auftauen wurde der flüssige Inhalt der Kühlfallen jeweils gesammelt und so schliesslich ca. 34 ml Wasser sowie 65 g getrocknetes pulverförmiges Cer(III)chlorid erhalten. Dieses wurde unter Inertagasatmosphäre in ein Vorratsgefäss übergeführt und unter Argonatmosphäre gelagert. Auch nach mehrmonatiger Lagerung bei Raumtemperatur wurde kein Aktivitätsverlust bei den nachfolgend beschriebenen *Grignard-*Reaktionen festgestellt.

### b) Vorkomplexierung des Ketons (typische, verallgemeinerbare Verfahrensweise):

36.4 g Cyclododecanon (0.2 mol) wurden zusammen mit 5 g wasserfreiem CeCl₃ (0.02 mol, 0.1 mol-Equiv.) bei Raumtemperatur in 100 ml absolutem THF suspendiert und unter Inertgasatmosphäre für 1 bis 2 Stunden intensiv gerührt, bis eine weissliche bis intensiv gelbe homogene Suspension von teilweise gelartiger Konsistenz erhalten wurde.

### 1. Vinyl-Grignard-Reaktion (typische, verallgemeinerbare Verfahrensweise):

### 1.1 1-Vinyl-1-Cyclododecanol aus Cyclododecanon:

Zu der durch Vorkomplexierung mit CeCl₃ aktivierten Suspension des Ketons wurden 320 ml einer 1-molaren Lösung von Vinylmagnesiumbromid in abs. THF (entspr. ca. 0.32 mol Vinylmagnesiumbromid, ca. 1.6 mol-Equiv.) innerhalb von ca. 5 min unter Rühren so zugegeben, dass unter zeitweiliger Einwirkung eines Kühlbades (Eisbad) die Innentemperatur im Reaktionsgefäss den Temperaturbereich von 35 - 40°C nicht überschritt. Nach Abklingen der exothermen Reaktion wird das nun gräulich-grüne Reaktionsgemisch noch für ca. 30 min bei 35 - 40°C gerührt, und der Reaktionsverlauf durch gaschromatographische Analysen verfolgt. Je nach Aktivität des eingesetzten Cer(III)chlorides zeigen diese bereits nach 15 Minuten in der Regel deutlich über 70% Eduktumsatz, wobei dann allenfalls noch unvollständig umgesetztes Keton durch erneute Zugabe von weiteren ca. 0.1 bis 0.2 mol-Equivalenten Vinylmagnesiumbromid zur Reaktion gebracht werden kann (über 80% Umsatz).

**Aufarbeitung**: Man lässt das Reaktionsgemisch auf Raumtemperatur abkühlen und giesst auf 1 1 Eiswasser, überschichtet mit dem Extraktionsmittel (Toluol oder TBME) und versetzt unter Rühren langsam mit einer ca. 5 - 10%igen wässrigen Salzsäurelösung, bis die schleim- oder gelartige Konsistenz des Gemisches verschwunden ist (ca. pH 3 oder darunter) und unter Auftreten einer gelben bis bräunlichen Färbung sich schliesslich eine klare Phasengrenze erkennen lässt. Man entfernt die wässrige Phase und wäscht die organische Phase zunächst mehrfach mit Wasser, dann mit Natriumhydrogencarbonat-Lösung oder auch ca. 5%iger NaOH-Lösung, anschliessend erneut mit Wasser, konz. wässriger NaCl-Lösung und trocknet schliesslich über Natriumsulfat bzw. Magnesiumsulfat. Nach Abdampfen des Extraktionsmittels unter vermindertem Druck ergaben sich als Rohprodukt 41.3 g 1-Vinyl-1-cyclododecanol (Rohausbeute 97 % d. Th., GC-Reinheit > 86 %, enthält ca. 12 - 14 % Cyclododecanon) als leicht gelblicher Festkörper. Dieser wurde entweder direkt für die nachfolgende Thermo-Isomerisierung eingesetzt oder zuvor entweder durch Kugelrohrdestillation am Hochvakuum und Umkristallisation aus Hexan/TBME (95:5, v:v) bzw. durch Chromatographie an Kieselgel (Hexan:TBME 9:1) gereinigt: Farbloser, wachsartiger Festkörper mit Schmp. 53°C.

¹H-NMR (300 MHz, CDCl₃): 5.98 (*dd*, *J* = 10.8, 17.4 Hz, 1 H), 5.20 (*dd, J* = 1.4, 17.4 Hz, 1 H), 5.01 (*dd, J* = 1.4, 10.8 Hz, 1 H), 1.9 - 1.2 (*m*, 23 H). ¹³C-NMR (75 MHz, CDCl₃) : 145.3 (*d*); 111.0 (*t*); 75.3 *(s);* 34.6 (2), 26.3 (2), 25.9, 22.5 (2), 22.1 (2), 19.5 (2) (6 *t*). EI-MS (GC/MS): 210.2 (2, *M*⁺•), 192.2 (50, *M -* 18), 77.7 (98), 67 (100), 55 (97).

In analoger Weise wurden auch beispielsweise die folgenden tertiären makrocyclischen Allylalkohole aus den entsprechenden Ketonen durch Addition von Vinylmagnesiumbromid hergestellt:

### 1.2. 1-Vinyl-1-cyclooctanol aus Cyclooctanon:

Ausbeute 94 %, GC-Reinheit > 90%

**¹H-NMR** (300 MHz, CDCl₃): 6.01 (*dd,* J = 10.8, 17.4 Hz, 1 H); 5.23 (*dd, J* = 1.3, 17.4 Hz, 1 H) ; 5.13 (*dd, J* = 1.3, 10.8 Hz, 1 H) ; 1.92 - 1.25 (*m*, 15 H). ¹³C-NMR (75 MHz, CDCl₃) : 145.8 (*d*); 111.1 (*t*); 75.1 (*s*); 36.2 (2), 28.1 (2), 24.6, 21.9 (2) (4 *t*).EI-MS (GC/MS) : 154 (2, *M*⁺•), 136 (100, *M* - H₂O).

### 1.3. 1-Vinyl-1-cyclodecanol aus Cyclodecanon:

¹H-NMR (300 MHz, CDCl₃): 5.99 (*dd, J =* 10.8, 17.4 Hz, 1 H); 5.21 (*dd, J =* 1.4, 17.4 Hz, 1 H) ; 5.01 (*dd, J =* 1.4, 10.8 Hz, 1 H); 1.85 - 1.25 (*m*, 19 H). ¹³C-NMR (75 MHz, CDCl₃) : 145.5 (*d*); 110.9 (*t*); 76.2 *(s);* 34.2 (2), 26.7, 26.1 (2), 23.5 (2), 21.1 (2) (4 *t*) .EI-MS (GC/MS) : 182 (1, *M*⁺•) , 164 (42, *M -* H₂O) , 149 (31), 135 (42), 79 (95), 68 (100), 55 (68).

### 1.4. 1-Vinyl-1-cycloundecanol aus Cycloundecanon:

Ausbeute 84 %.

¹H-NMR (300 MHz, CDCl₃): 5.98 (*dd, J =* 10.8, 17.4 Hz, 1 H); 5.20 (*dd, J =* 1.4, 17.4 Hz, 1 H); 5.01 (*dd, J =* 1.3, 10.8 Hz, 1 H); 1.76 - 1.20 (*m*, 21 H). ¹³C-NMR (75 MHz, CDCl₃): 145.4 (*d*); 111.1 (*t*); 75.8 *(s);* 36.2, 27.0, 25.9, 25.4, 21.1 (5 *t*, je 2 CH₂). EI-MS (GC/MS): 196.1 (1, *M*⁺•) , 178 (15, *M -* H₂O) , 169 (18) , 149 (17), 135 (20), 111 (55), 97 (80), 83 (100), 70 (95), 55 (100).

### 1.5. 1-Vinyl-1-cyclotridecanol aus Cyclotridecanon:

Ausbeute 84 %, Gehalt gemäss GC 92%.

¹H-NMR (300 MHz, CDCl₃): 5.98 (*dd, J* = 10.8, 17.4 Hz, 1 H); 5.21 (*dd, J =* 1.4, 17.4 Hz, 1 H) ; 5.01 (*dd, J =* 1.4, 10.8 Hz, 1 H); 1.65 - 1.2 (*m*, 25 H). ¹³C-NMR (75 MHz, CDCl₃) : 145.4 (*d*); 111.1 (*t*); 75.1 (*s*); 37.4, 27.8, 26.6, 25.4, 25.3, 20.9 (5 *t*). EI-MS (GC/MS): 224 (1, *M*⁺•.), 206 (100, *M* - H₂O).

### 1.6. 1-Vinyl-1-cyclotetradecanol aus Cyclotetradecanon:

Ausbeute 98%.

¹H-NMR (300 MHz, CDCl₃): 5.99 (*dd, J =* 10.8, 17.4 Hz, 1 H); 5.22 (*dd, J =* 1.4, 17.4 Hz, 1 H) ; 5.03 (*dd, J =* 1.4, 10.8 Hz, 1 H) ; 1.6 - 1.2 (*m*, 27 H). ¹³C-NMR (75 MHz, CDCl₃): 145.4 (*d*); 111.3 (*t*); 75.0 *(s);* 37.2 (2), 26.43 (2), 26.38, 25.9, 24.0, 23.5, 20.3 (7 *t*). EI-MS (GC/MS): 224 (1, *M*⁺•) , 206 (100, *M -* H₂O).

### 1.7. 1-Vinyl-1-cyclopentadecanol aus Cyclopentadecanon:

Ausbeute 92%.

¹H-NMR (300 MHz, CDCl₃) : 5.97 *(dd, J* = 10.8, 17.4 Hz, 1 H) ; 5.22 (*dd, J =* 1.4, 17.4 Hz, 1 H); 5.03 (*dd, J =* 1.4, 10.8 Hz, 1 H); 1.6 - 1.2 (*m*, 29 H). ¹³C-NMR (75 MHz, CDCl₃) : 145.4 (*d*); 111.3 (*t*); 75.0 (*s*); 37.2 (2), 26.43 (2), 26.38, 25.9, 24.0, 23.5, 20.3 (7 *t*). EI-MS (GC/MS): 252.1 (1, *M*⁺•.), 234 (10, *M* - H₂O)

### 1.8. (syn/anti)-2-Methyl-1-vinyl-1-cyclododecanol aus (R/S)-2-Methylcyclodecanon:

Ausbeute 98%.

¹H-NMR (300 MHz, CDCl₃): 5.93/5.88 (*dd, J* = 10.8, 17.3 Hz, 1 H); 5.24/5.23 (*dd, J* = 1.6, 17.3 Hz, 1 H); 5.08/5.06 (*dd*, *J* = 1.6, 10.8 Hz, 1 H); 2.1 - 1.1 (*m*, 22 H), 0.85/0.81 (*d*, *J* = 6.6 Hz, 3 H). ¹³C-NMR (75 MHz, CDCl₃): 144.7/142.4 *(d)*; 112.2/111.0 (*t*); 78.0/77.9 *(s);* 38.4/35.9 (*t*); 35.5/34.4 (*d*); 28.7, 26.8, 26.5, 26.3 (2), 25.0, 24.8, 23.4, 23.1, 23.0, 22.9, 22.8 (2), 22.7, 22.2, 22.1, 20.4, 18.4 (18 *t*); 14.6/13.6 (*q*). EI-MS (GC/MS): 224.1 (7, *M*⁺•) , 209 (9, *M* - 15), 206 (6, *M -* 18).

### 1.9. (syn/anti)-3-Methyl-1-vinyl-1-cyclopentadecanol aus rac. 3-Methylcyclopentadecanon:

Ausbeute 93%

¹H-NMR (300 MHz, CDCl₃) von **A**: 5.93 (*dd, J* = 10.7, 17.3 Hz, 1 H); 5.18 (*dd, J* = 1.3, 17.3 Hz, 1 H); 5.0 (*dd*, *J* = 1.3, 10.7 Hz, 1 H); 1.8 - 1.0 (*m*, 28 H), 0.99 (*d*, *J* = 6.6 Hz, 3 H); von **B**: 5.97 (*dd*, *J* = 10.8, 17.4 Hz, 1 H); 5.21 *(dd, J* = 1.4, 17.4 Hz, 1 H) ; 5.0 (*dd, J* = 1.4, 10.8 Hz, 1 H) ; 1.8 - 1.0 (*m*, 28 H), 0.88 (d, *J* = 6.5 Hz, 3 H). ¹³C-NMR (75 MHz, CDCl₃) von **A** (Hauptisomer): 146.0 *(d)*; 110.9 *(t);* 75.7 *(s) ;* 46.0, 38.9, 37.2 (3 *t*) , 27.0 (*d*) 22.2 (*q*) , 26.6 (*t*)*;* von **B**: 146.0 (*d*)*;* 110.9 (*t*) ; 75.7 *(s) ;* 46.0, 38.9, 37.2 (3 *t*), 27.0 (*d*) 22.2 (*q*) , 26.6 (*t*); von **A** oder **B**: 27.4, 27.2, 27.1, 27.0, 26.9, 26.7, 26.6, 26.4, 26.3, 26.2, 26.0, 25.9 (2), 25.8, 25.0, 24.9 (16 *t*); von **A**: 22.2 (*q*) , 22.6 (*t*); von **B:** 21.7 (*q*), 21.2 (*t*).

EI-MS (GC/MS) von **A**: 266 (2, *M*⁺•·) , 248 (28, *M -* H₂O) , 233 (7), 219 (11), 121 (40), 107 (55), 94 (58), 67 (83) , 55 (100); von **B:** 266 (4, *M*⁺•) , 248 (100, *M -* H₂O) , 233 (12), 219 (16), 121 (30),107 (42), 67 (55) , 55 (100).

### 1.10. (syn/anti)-2-Ethyl-1-vinyl-1-cyclododecanol aus (R/S)-2-Ethylcyclodecanon:

Ausbeute 98%.

¹H-NMR (300 MHz, CDCl₃) des Diastereoisomerengemisches: 5.93/5.88 (*dd, J =* 10.8, 17.3 Hz, 1 H); 5.24/5.23 (*dd*, *J* = 1.6, 17.3 Hz, 1 H); 5.08/5.06 (*dd, J* = 1.6, 10.8 Hz, 1 H); 2.1 - 1.1 (*m*, 24 H), 0.97/0.91 (2t, *J* = 7.3 Hz, 3 H) . ¹³C-NMR (75 MHz, CDCl₃): 144.7/143.0 (*d*); 111.7/110.7 *(t);* 78.7/78.6 *(s);* 43.0/41.4 *(d)*; 39.2, 37.0, 28.2, 26.7, 26.6, 25.9, 25.7 (2), 24.8, 23.8, 23.7, 23.6, 23.5, 23.4, 23.2, 23.1, 23.0, 22.8, 22.4, 22.2, 20.6, 18.6 (22 *t*); 14.7/13.2 *(q)*.

### 2. Grignard-Reaktionen mit Alkyl-substituierten Vinylhalogeniden:

### a) 1-Propenyl-Grignard-Reaktionen

### 2.1. (E/Z)-1-(1-Propen-1-yl)-cyclododecanol:

Unter Rühren wurden zu 18.2 g Cyclododecanon (0.1 mol), das zuvor analog zu Beispiel Ib der obigen Beschreibung mit 2.5 g CeCl₃ (10 mmol, 0.1 mol-Equiv.) vorkomplexiert wurde, via Kanüle die Lösung des zuvor aus 3.9 g Magnesium (0.16 mol) und 20.6 g 1-Brom-1-propen (*E*/*Z*-Gemisch, 0.17 mol) in 160 ml absolutem THF nach den üblichen Methoden zubereiteten *Grignard*-Reagenz' zugegeben, wobei in entsprechender Weise wie in Beispiel 1.1 beschrieben vorgegangen wurde. Rohausbeute an (*E*/*Z*)-1-(1-Propenylcyclododecanol): 21 g (94 %), wachsartiger Festkörper, GC-Reinheit 92 % (enthält ca. 6 % Cyclododecanon). Reinigung durch Umkristallisation aus Hexan:Ether 95:5 (v:v). Trennung des *E-* und Z-Isomers durch Säulenchromatographie (Kieselgel, Hexan, Ether 9:1), wobei das weniger polare Z-Isomer (weisser Festkörper) zuerst eluiert wurde:
***Z*-Isomer:** ¹H-NMR (300 MHz, CDCl₃) : 5.50 - 5.36 (*m*, 2 H; Analyse bei 600 MHz zeigt 5.46 (*dq, J =* 11.8 Hz, 7.0 Hz), 5.39 *dq* (*J =* 11.8 Hz, 1.6 Hz)), 1.87 (*dd, J* = 7 Hz, 1.6 Hz, 3 H), 1.75 - 1.55 (*m,* 4 H), 1.49 - 1.2 (*m*, 19 H). ¹³C -NMR (75 MHz, CDCl₃): 136.4 *(d);* 125:8 *(d);* 76.1 *(s);* 35.8 (2) ; 26.5 (2), 26.1, 22.6 (2), 22.3 (2), 19.7 (2) (6 *t*); 14.5 *(q).*
***E*-Isomer** (farbloser, wachsartiger Festkörper): ¹H-NMR (300 MHz, CDCl₃): 5.68 - 5.55 (*m*, 2H; Analyse bei 600 MHz zeigt 5.64 (*dq, J =* 15.6, 6 Hz), 5.59 (*dq, J =* 15.6, 1.1 Hz)), 1.69 (*dd, J =* 6, 1 Hz, 3 H), 1.68 - 1.22 (*m,* 23 H). ¹³C-NMR (75 MHz, CDCl₃): 138.4 *(d);* 122.2 *(d);* 74.9 *(s);* 35.0 (2) ; 26.5 (2), 25.9, 22.6 (2), 22.2 (2), 19.6 (2) (6 *t*) ; 17.7 (*q*).

In analoger Weise wurden auch beispielsweise die folgenden tertiären makrocyclischen Allylalkohole aus dem entsprechenden Keton durch Addition von (*E*/*Z*)-1-(1-Propenyl)magnesiumbromid hergestellt:

### 2.2. (E/Z)-1-(1-Propenyl)cyclotridecanol aus Cyclotridecanon:

Ausbeute 82%.

***Z*-Isomer** (weisser Festkörper): **¹**H-NMR (300 MHz, CDCl₃): 5.50 - 5.39 (*m*, 2 H), 1.87 (*dd, J =* 6.5, 1.8 Hz, 3 H), 1.70 - 1.60 (*m,* 4 H), 1.35 (br. *s*-artiges *m,* 21 H). ¹³C-NMR (75 MHz, CDCl₃): 136.5 *(d);* 125.7 (*d*); 76.0 *(s);* 38.8 (2); 27.8 (2), 26.6 (2), 25.4 (4), 21.1 (6 *t*); 14.3 (*q*).

***E*-Isomer** (farbloses, viskoses Öl, Hauptisomer): ¹H-NMR (300 MHz, CDCl₃) : 5.65 - 5.55 (*m,* 2H) , 1.69 (*d, J* = 5, 3 H), 1.-68 - 1.46 (*m,* 4 H), 1.35 (br. *s*-artiges m, 21 H). ¹³C-NMR (75 MHz, CDCl₃) : 138.2 (*d*); 122.2 (*d*); 74.6 *(s);* 37.8 (2); 27.9 (2), 26.7 (2), 25.5 (2) 25.4 (2), 21.0 (2), (6 *t*) ; 17.7 (*q*).

### 2.3. (E/Z)-1-(1-Propen-1-yl)cyclotetradecanol aus Cyclotetradecanon:

Ausbeute 93%.

***Z*-Isomer** (weisser Festkörper): ¹H-NMR (300 MHz, CDCl₃): 5.52 - 5.39 (*m*, 2 H; Analyse bei 600 MHz zeigt 5.46 (*dq, J* = 12, 6.7 Hz), 5.43 *dq* (*J* = 12, 1.3 Hz)), 1.87 (*dd, J* = 5.6 Hz, 1.9 Hz, 3 H), 1.68 - 1.51 (*m*, 4 H), 1.39 - 1.18 (*m*, 21 H). ¹³C-NMR (75 MHz, CDCl₃) : 136.6 (*d*); 125.7 (*d*); 75.9 *(s)*; 38.8 (2) ; 27.8 (2), 26.5 (2), 26.4, 26.0 (2), 23.5 (2), 20.4 (2) (7 *t*); 14.3 (*q*).

***E*-Isomer** (Hauptisomer, farbloses, viskoses Öl, das beim Stehenlassen allmählich zu einem Festkörper erstarrt): ¹H-NMR (300 MHz, CDCl₃) : 5.71 - 5.56 (*m*, 2H; Analyse bei 600 MHz zeigt 5.65 (*dq*, *J* = 16 Hz, 6 Hz), 5.59 *dq* (*J* = 16 Hz, 1.1 Hz)), 1.70 (*d*, *J* = 5 Hz, 3 H), 1.63 - 1.18 (*m,* 27 H). ¹³C -NMR (75 MHz, CDCl₃): 138.5 (*d*); 122.4 (*d*); 74.6 *(s);* 37.5 (2); 26.5 (2), 26.4, 26.0 (2), 24.0 (2) 23.5 (2) , 20.4 (2) , (7 *t*); 17.7 (*q*).

### 2.4. (E/Z)-1-(1-Propen-1-yl)-cyclopentadecanol aus Cyclopentadecanon:

Ausbeute 90%.

***E*/*Z*-Isomerengemisch:** ¹H-NMR (300 MHz, CDCl₃): 5.68 - 5.44 (*m*)/5.50 - 5.39 (*m*, 2 H); 1.87 (*d, J* = 5.8 Hz)/ 1.69 (*d, J* = 5.2 Hz, 3 H), 1.67 - 1.20 (*m*, 29 H). ¹³C -NMR (75 MHz, CDCl₃): 138.4/136.5 *(d);* 125.8/122.5 (*d*); 75.9/74.6 *(s);* 39.5/38.8; 26.9, 26.6 (3), 26.24, 26.20, 21.9, 21.8 ; 17.7/14.3 (*q*).

### b) 2-Propenyl-Grignard-Reaktionen (typische, analoge Verfahrensweise):

### 2.5. 1-(1-Methylethenyl)-cyclododecanol aus Cyclododecanon:

14.5 g Cyclododecanon (80 mmol), Vorkomplexierung mit 1 g CeCl₃ (4 mmol = 0.05 mol-Equiv.); ca. 100 mmol 1-Propen-2-ylmagnesiumbromid (frisch zubereitet aus 2.43 g Magnesium und 14 g 2-Brom-1-propen in THF). Nach 2 h gemäss GC noch ca. 15 % Edukt. Nach üblicher Aufarbeitung und Kugelrohrdestillation im Hochvakuum wurden 16.8 g (93 %) farbloses, viskoses Öl erhalten, das beim Stehenlassen allmählich erstarrte (enthält gemäss GC noch 15 % Cyclododecanon). Nach zweimaligem Umkristallisieren aus Hexan bei -15°C ergaben sich 12.9 g (72 %) eines weissen kristallinen Festkörpers.

¹H-NMR (300 MHz, CDCl₃): 4.84 - 4.82 (*m*, 2 H); 1.79 (*d*, 0.6 Hz, 3H), 1.63 (*m*_{c}, 4 H); 1.4 - 1.2 (*m*, 19 H). ¹³C-NMR (75 MHz, CDCl₃) : 150.6 *(s)*; 110.4 (*t*); 76.8 *(s)*; 32.8 (2), 26.5 (2), 26.1, 22.5 (2), 22.2 (2), 19.9 (2) (6 *t*); 18.8 (*q*).

EI-MS (GC/MS) : 224.1 (4, *M*⁺•), 206. 1 (28, *M -* H₂O) , 55.0 (100).

### 2.6. 1-(1-Methylethenyl)-cycloundecanol aus Cycloundecanon:

Ausbeute 88%.

¹H-NMR (300 MHz, CDCl₃) : 4.83 (*d*, *J* = 16.4 Hz, 2 H); 1.78 (*d*, 0.6 Hz, 3 H), 1.74 - 1.70 (*m*, 4 H); 1.64 - 1.2 (*m*, 17 H). ¹³C-NMR (75 MHz, CDCl₃) : 150.6 (*s*); 110.1 (*t*); 77.3 (*s*); 34.1 (2), 27.1 (2) , 26.1 (2) , 25.4 (2) , 21.5 (2) , (5 *t*); 18.8 (*q*)*.*

EI-MS (GC/MS): 210.1 (1, *M*⁺•), 192.0 (95, M - H₂O), 148.8 (100).

### c) Grignard-Reaktionen mit Alkyl-disubstituierten Vinylhalogeniden:

### 2.7. 1-(2-Methyl-1-propenyl)cyclododecanol aus Cyclododecanon:

3.0 g Cyclododecanon (16.5 mmol), Vorkomplexierung mit 1 g CeCl₃ (4 mmol = 0.25 mol-Equiv.); ca. 25 mmol 2-Methyl-1-propen-1-ylmagnesiumbromid (frisch zubereitet aus 0.6 g Magnesium und 3.5 g 1-Brom-2-methyl-1-propen (Isocrotylbromid) in THF). Nach 2 h gemäss GC noch ca. 3 % Edukt. Nach üblicher Aufarbeitung und Kugelrohrdestillation im Hochvakuum wurden 2.9 g (74 %) farbloses, viskoses Öl erhalten, das beim Stehenlassen allmählich wachsartig erstarrte.

¹H-NMR (300 MHz, CDCl₃): 5.22 (br. *s*-artiges m, 1 H); 1.87 (*d*, *J* = 1.2 Hz, 3 H), 1.69 (*d*, *J* = 1.2 Hz 3 H) ; 1.63 - 1.5 (*m*, 4 H); 1.48 - 1.2 (*m*, 19 H). ¹³C-NMR (75 MHz, CDCl₃) : 134.2 (*s*); 130.7 (*d*) *;* 75.1 (*s*); 36.0 (2) (*t*) , 27.2 (*q*) ; 26.4 (2), 25.9, 22.5 (2) , 22.2 (2), 19.6 (2) (5 *t*); 18.9 (*q*).

EI-MS (GC/MS): 238.1 (8, *M*⁺•) , 220.1 (45, *M* - H₂O) , 96.0 (100).

### 2.8. (E/Z)-1-(2-Buten-2-yl)cyclododecanol aus Cyclododecanon:

9.1 g Cyclododecanon (50 mmol), Vorkomplexierung mit 2 g CeCl₃ (8 mmol = 0.16 mol-Equiv.); ca. 80 mmol (*E*/*Z*)-2-Buten-2-ylmagnesiumbromid (frisch zubereitet aus 2.0 g Magnesium und 10.8 g 1-Brom-2-methyl-1-propen in THF). Nach 2 h gemäss GC noch ca. 30 % Edukt neben 2 Hauptprodukten (31 bzw. 15 %). Nach üblicher Aufarbeitung wurden 11.3 g (95 %) eines leicht gelblichen wachsartigen Festkörpers erhalten. Zweimaliges Umkristallisieren aus Hexan bei -20°C ergibt 4.3 g (36 %) farblose Kristalle eines E/Z-Isomerengemisches (ca. 4:1).

Trennung der Isomeren durch Säulenchromatographie an Kieselgel (Hexan/TBME 94:6):
¹H-NMR (300 MHz, CDCl₃) Isomer **A** (zuerst eluiert, farblose Kristalle): 5.37 (*dq*, *J* = 7.3, 1.3 Hz, 1 H), 1.84 (*dd*, *J* = 7.3, 1.3 Hz, 3 H), 1.72 - 1.70 (*m*, 7 H), 1.45 - 1.25 (*m*, 19 H). ¹³C-NMR (75 MHz, CDCl₃) : 140.9 *(s)*, 122.8 (*d*), 77.8 *(s)* , 34.6 (2), 26.4 (2) , 26.0 (3 *t*), 23.2 (*q*), 22.4 (2) , 22.1 (2) , 19.6 (3 *t*), 15.1 (*q*).
¹H-NMR (300 MHz, CDCl₃) Isomer **B** (später eluiert, farblose Kristalle) : 5.43 (*m*_{c}, 1 H) , 1.67 (*m*, 3 H), 1.62 - 1.55 (*m,* 7 H), 1.45 - 1. 1 (*m*, 19 H). ¹³C-NMR (75 MHz, CDCl₃) : 140.8 *(s) ,* 118.2 *(d),* 77.1 *(s),* 32.7 (2) , 26.3 (2) , 26.0, 22.4 (2) , 22.1 (2) (6 *t*), 13 .3 (*q*), 11.6 (*q*).

### 2.9. (E/Z)-2-Methyl-1-(1-propen-1-yl)cyclododecanol aus 2-Methylcyclododecanon:

**Isomerengemisch:** charakteristische Signale im ¹³C-NMR (75 MHz, CDCl₃): 137.4, 135.4, 135.1, 133.2, 125.7 ,124.8, 122.8, 121.4 (8 *d*); 79.6, 79.2, 77.3, 77.2 (4 *s*); 37.1, 36.2, 35.6, 34.8 (4 *d*); 14.5, 14.4, 13.9, 13.5 (4 *q*). EI-MS (GC/MS): 238.1 (*M*⁺•).

### 3. Addition von cyclischen Alken-1-yl-Derivaten

### 3.1. 1-(1-Cyc1ohexenyl)cyclododecan-1-ol

Herstellung mit 1-Chlor-l-cyclohexen gemäss Literatur (vgl. Marson et al. J. Org. Chem. 1993, 58, 5944 -5951, spez. S. 5948; und Adam, Synthesis 1994, 176 - 180).

¹H-NMR (300 MHz, CDCl₃): 5.62 - 5.59 (*m*, 1 H), 2.11 - 2. 01 (*m*, 4 H), 1. 66 - 1.52 (*m*, 8 H) , 1. 37 1.22 (*m*, 19 H) . ¹³C-NMR (75 MHz, CDCl₃): 142.5 (*s*), 121.1 (*d*), 76.8 (*s*), 32.8 (2), 26.5 (2), 26.2, 25.4, 23.9, 23.2, 22.5 (2), 22.2 (2), 19.9 (2).

### 4. Addition von Alkinyl-Derivaten

### 4.1. 1-Ethinylcyclododecanol aus Cyclododecanon:

5.5 g Cyclododecanon (30 mmol), Vorkomplexierung mit 1 g CeCl₃ (4 mmol = 0.13 mol-Equiv.); ca. 60 mmol Ethinylmagnesiumbromid (120 ml einer 0.5 M Lösung in THF). Nach 2 h gemäss GC noch ca. 2 - 5 % Edukt und ca. 90 % eines Hauptproduktes. Nach üblicher Aufarbeitung wurden 5.3 g (85 %) eines leicht gelblichen Festkörpers erhalten. Umkristallisieren aus Hexan/TBME (9:1, v:v) bei 4°C ergibt 4.5 g (72 %) farblose, transparente Kristalle. Ebenso wurde Ethinylcyclododecanol (techn. Qualität, Fa. GIVAUDAN) durch Destillation im HV und Kristallisation aus Hexan/TBME 9:1 (GC-Gehalt > 97 %) gereinigt.

¹H-NMR (300 MHz, CDCl₃): 2.44 (*s*, 1 H), 2.12 (*s*, 1 H), 1.91 - 1. 80 (*m*, 2 H), 1.75 - 1.63 (*m*, 2 H), 1.60 - 1.2 (*m*, 18 H). ¹³C-NMR (75 MHz, CDCl₃) : 88.5 *(s)* , 71.4 (*d*), 70.8 (*s*), 35.8 (2), 26.1 (2), 25.9, 22.5 (2), 22.2 (2), 19.6 (2) (6 *t*).

EI-MS: 208 (1, *M*⁺•), 190 (15) , 175 (4) , 161 (10) , 147 (20) , 133 (25), 119 (30), 105 (50), 93 (60), 91 (100), 79 (75), 67 (35), 55 (32).

### 4.2. 1-Ethinylcyclotridecanol aus Cyclotridecanon:

¹H-NMR (300 MHz, CDCl₃): 2.44 (*s*, 1 H), 1.93 (*s*, 1 H), 1.83 - 1.67 (*m,* 4 H), 1.53 - 1.34 (*m,* 20 H). ¹³C-NMR (75 MHz, CDCl₃): 88.2 *(s),* 71.3 (*d*), 70.7 *(s),* 38.8 (2), 27.2 (2), 26.4 (2), 25.4 (2) , 25.3 (2), 21.1 (2) (6 *t*) .EI-MS: 222 (1, *M*⁺•), 221 (1, M-1), 207(5), 196 (6), 179 (5), 165 (8) 161 (7), 151 (15) 137 (20), 123 (36), 109 (45), 97 (50), 68 (100) 55 (99).

### 4.3. 1-(1-Propinyl)cyclododecanol aus Cyclododecanon:

3.64 g Cyclododecanon (20 mmol), Vorkomplexierung mit 2 mmol CeCl₃ in 30 ml THF, 30 mmol 1-Propinylmagnesiumbromid (entspr. 60 ml einer ca. 0.5 M Lösung in THF), nur leichte Exothermie. Nach der üblichen Aufarbeitung 3.8 g (85 %) gelbliches Öl (GC-Gehalt > 92 %, das beim Stehenlassen kristallisiert. Reinigung durch Kugelrohrdestillation: 3.6 g (81 %) farbloses Öl, das kristallin erstarrt.

¹H-NMR (300 MHz, CDCl₃): 1.83 (*s*, 3 H), 1.81 - 1.48 (*m*, 5 H), 1.35 (br. *s*-artiges *m*, 18 H). ¹³C-NMR (75 MHz, CDCl₃): 83.8 (*s*), 79.1 (*s*), 70.8 (*s*), 36.2 (2), 26.0 (2), 25.8, 22.4 (2), 22.2 (2), 19.7 (2) (6 *t*) 3.3 (*q*). EI-MS (GC/MS): 224.1 (0.5, M+2), 223.1 (2, *M*+1), 222.1 (3, M⁺•), 204.1 (30, M - 18) , 189.0 (8) , 175 (9), 161 (18), 147 (32), 98 (62), 95 (90), 91 (100), 83 (85), 67 (95), 55 (98).

### 4.4. 1-(1-Propinyl)cyclotridecanol aus Cyclotridecanon:

¹H-NMR: 1.83 (*s*, 3 H), 1.78 - 1.62 (*m*, 5 H), 1.50 - 1.26 (br. *s-*artiges *m*, 20 H).

¹³C-N_{MR} (75 MHz, CDCl₃) : 83.7 *(s),* 79.1 (*s*), 70.9 (*s*), 39.2 (2), 27.3 (2), 26.5(2), 25.4 (2), 25.3 (2), 21.3 (2) (6 *t*) 3.3 (*q*). EI-MS (GC/MS): 236 (2, M⁺•), 235 (1, *M*-1), 221 (22), 207 (5), 196 (10), 179 (11) , 165 (15) , 151 (22), 137 (35) , 123 (45) , 109 (51), 95 (75), 82.5 (100), 67.8 (62), 56 (98).

### II. Thermo-Isomerisierungen

### Apparatur und allgemeine Vorgehensweise:

Der tertiäre cyclische Alkohol wurde jeweils in einem Kugelrohrkolben (50 bzw. 100 ml, mit zwei gegenüberliegenden Schliffhälsen) zusammen mit einem kleinen Magnetrührer vorgelegt. Nach Anbringen einer Inertgas-Zuleitung (Edelstahl-Kapillare eingeschmolzen in Normschliffaufsatz) am hinteren Schliffhals wurde der Kolben mit dem vorderen Schliff an das leicht geneigt angeordnete Reaktorgefäss (Quartzglas-Reaktor, Innendurchmesser 25 bzw. 40 mm, Länge 40 cm, beheizt mit *Thermolyne* Tube Furnace, 35 cm) angeschlossen. Am gegenüberliegenden Ende des Reaktorgefässes befindet sich eine Kühlfalle (Kühlmedium Flüssigstickstoff bzw. Trockeneis/Aceton), die mit einer Hochvakuumpumpen-Einheit verbunden ist. Nach Äquilibrieren der Reaktortemperatur auf 650 - 660°C (gemessen in der Mitte an der Raktorgefässaussenwand), Evakuieren der gesamten Apparatur und Einstellung eines Druckes von ca. 1 mbar (1 hPa) wurde der Kolben mit dem vorgelegten Alkohol im Luftbad (Heizmantel von modifiziertem *Büchi*-Kugelrohrofen) aufgeheizt und so das Edukt unter Rühren verdampft und durch das Reaktorgefäss destilliert. Dabei wurde mit einem Präzisionsnadelventil ein schwacher Inertgasstrom (Stickstoff, gemäss *Vögtlin* Typ V100-Flowmeter 1 - 5 1/h) eingestellt und via Kapillare durch die Apparatur geleitet. Am Ausgang des Reaktors begann jeweils kurz darauf ein farbloses Öl zu kondensieren, das in einem Auffanggefäss unterhalb der Kühlfalle gesammelt wurde und dort teilweise erstarrte. Nach ca. 15 - 45 min war das Ausgangsmaterial meist nahezu rückstandsfrei verdampft. Nach Abkühlen der Apparatur, Spülen mit Inertgas wurde das Kondensat mit Hexan aus der Kühlfalle ausgewaschen. Alle angegebenen Ausbeuten sind typische Durchschnittswerte in der Regel aus jeweils zumindests drei separat durchgeführten Thermo-Isomerisierungen.

### A. Unsubstituierte monocyclische Systeme:

### 1.1 Cyclotetradecanon aus 1-Vinylcyclododecanol:

5 g (23.8 mmol) 1-Vinylcyclododecanol, Quartzreaktor 25/400 mm, Temperatur 660°C ± 10, Verdampfung bei 125 - 135°C (Luftbadtemperatur) in ca. 30 min. Stickstoffstrom ca. 1 - 2 1/h, Vakuum 4 - 6 mbar. Das ölige Kondensat begann bereits in der Kühlfalle zu kristallisieren. Es wurden 4.4 g (88% Rohausbeute) eines wachsartigen Festkörpers erhalten, der gemäss GC- und GC/MS-Analysen neben 80 - 85 % der Hauptkomponente noch zu ca. 5 % Cyclododecanon sowie ca. 5 - 10 % weitere Komponenten enthält, bei denen es sich aufgrund der gefundenen Molekülmassen um Dehydrierungsprodukte handelt. Durch zweimaliges Umkristallisieren aus Hexan bei 0° und -20°C wurden 3.6 g (72 %) Cyclotetradecanon (GC-Reinheit > 98 %) als farblose Nadeln mit Smp. 56°C erhalten.

¹H-NMR (300 MHz, CDCl₃): 2.44 (*t*-artiges zentr. *m*, 4 H), 1.71 - 1.32 (*m*, 4 H), 1.29 (br., *s*-artiges *m*, 18 H). ¹³C-NMR (75 MHz, CDCl₃): 212.2 *(s),* 40.9, 26.1, 25.8, 25.35, 25.30, 24.5, 22.9 (7 *t*, je 2 CH₂). EI-MS (GC/MS): 211.2 (28), 210.2 (27, *M*⁺•**,** 152.1 (18), 125.1 (22), 111.1 (28), 96.1 (47), 71(96), 55 (100).

In analoger Weise wurden hergestellt:

### 1.2. Cyclodecanon aus 1-Vinylcyclooctanol.

Rohausbeutet 87%.

¹H-NMR (300 MHz, CDCl₃): 2.44 (*t*-artiges zentr. *m*, 4 H), 1.71 - 1.32 (*m*, 4 H), 1.29 (br., *s*-artiges m, 10 H). ¹³C-NMR (75 MHz, CDCl₃): 212.2 *(s)*, 40.9, 26.1, 25.8, 25.35, 25.30, 24.5, 22.9 (7 *t*, je 2 CH₂) . EI-MS (GC/MS): 156.1 (8) , 155.1 (58) , 154.0 (75, *M*⁺•), 125.0 (65), 110.9 (100).

### 1.3. Cyclododecanon aus 1-Vinylcyclodecanol.

0.5 g (2.7 mmol) 1-Vinyl-1-cyclodecanol (vgl. I.1.3, enthält noch ca. 8 - 10 % Cyclodecanon), Quartzreaktor 25/400 mm, Temperatur 670°C ± 10, Verdampfung bei 95 - 100°C (Luftbadtemperatur) in ca. 10 min. Stickstoffstrom ca. 2 - 2.5 1/h, Vakuum 5 - 6 mbar. Es wurden 0.45 g (90% Rohausbeute) eines reinweissen Kondensates erhalten, das teilweise in feinen Nädelchen kristallisierte. Gemäss GC- und GC/MS-Analysen enthält das Gemisch als Hauptkomponente zu ca. 70 % Cyclododecanon, neben 6 - 8 % Cyclodecanon und 2 - 3 % Ausgangsmaterial. Durch chromatographische Trennung des Gemisches an Kieselgel (Hexan/TBME 97:3) wurden 0.26 g Cyclododecanon (52 % isol. Ausb.) erhalten, das identische Retentionszeiten und spektroskopische Eigenschaften wie die kommerziell erhältliche Verbindung (Fluka) aufweist.

¹H-NMR (300 MHz, CDCl₃) : 2.46 (*m*_{c}, 4 H), 1.71 (*m*_{c}, 4 H), 1.30 (br., *s*-artiges m, 14 H). ¹³C-NMR (75 MHz, CDCl₃): 214.8 (*s*), 40.4 (2), 24.8 (2), 24.7 (2), 24.3 (2), 22.6 (2), 22.4 (6 *t*). EI-MS (GC/MS): 183.1 (4), 182.1 (10, *M*⁺•), 139.0 (8), 125.0 (13), 111.0 (25), 98.0 (33), 71(35), 55.0 (100).

### 1.4. Cyclotridecanon aus 1-Vinylcycloundecanol.

¹H-NMR (300 MHz, CDCl₃) : 2.44 (*m*_{c}, 4 H), 1.67 (*m*_{c}, 4 H), 1.31 - 1.2 (br., *s*-artiges m, 16 H). ¹³C-NMR (300 MHz, CDCl₃): 212.7 (*s*), 41.9 (2) , 26.4 (2) , 25.7 (2) , 25.6 (2) , 24.4 (2), 23.2 (6 *t*). EI-MS (GC/MS): 198.2 (5) , 197.1 (33) , 196.0 (40, *M*⁺•), 153.0 (15), 149.0 (25), 138 (30) 125.0 (35), 111.0 (35), 98.0 (33), 71(35), 55.0 (100).

### 1.5. Cyclopentadecanon aus 1-Vinylcyclotridecanol.

**¹H-NMR** (300 MHz, CDCl₃) : 2.41 (*t*-artiges m_{c}, 4 H), 1.64 (*m*_{c}, 4 H), 1.37 - 1.30 (br., *s*-artiges *m*, 20 H). ¹³C-NMR (75 MHz, CDCl₃): 212.5 (*s*), 42.0 (2), 27.5 (2), 26.70 (2), 26.66 (2), 26.4 (2), 26.2 (2), 23.4 (2) (7 *t*). EI-MS (GC/MS): 226.1 (2), 225.1 (8) , 224.1 (13, *M*⁺•) , 166.1 (8) , 149.0 (8) , 135 (10) 125.0 (15), 111.0 (18), 98.0 (21), 71(66), 55.0 (100).

### 1.6. Cyclohexadecanon aus 1-Vinylcyclotetradecanol.

¹H-NMR (300 MHz, CDCl₃): 2.40 (*m*_{c}**,** 4 H), 1.64 (*m*_{c}, 4 H), 1.32 - 1.25 (br., *s*-artiges *m*, 22 H). ¹³C-NMR (75 MHz, CDCl₃): 212.2 *(s),* 41.9, 27.5, 27.1, 26.9, 26.5 (2) , 26.4, 23.3 (8 *t*). EI-MS (GC/MS): 239.2 (10, *M*+1) , 238.1 (43, *M*⁺•) , 223.1 (8) , 209.1 (5) , 163.0 (12), 149 (18), 135 (28), 125.0 (53), 111.0 (45), 98.0 (76), 82.0 (72), 71.0(100), 58.0 (95), 55.0 (99).

### 1.7. Cycloheptadecanon aus 1-Vinylcyclopentadecanol.

¹H-NMR (300 MHz, CDCl₃) : 2.39 (*m*_{c}, 4 H), 1.62 (*m_{c}* 4 H), 1.32 - 1.25 (br., *s*-artiges *m*, 24 H). ¹³C-NMR (75 MHz, CDCl₃): 212.0 *(s),* 42.2, 28.1, 27.7, 27.5, 27.2, 27.1, 26.8, 23.6, (16 *t*). EI-MS (GC/MS): 253.2 (4, *M*+1), 252.1 (20, *M*⁺•), 237.1 (3), 234.1 (4) , 223.1 (4) , 210.1 (5) , 194.1 (6) , 163.0 (7) , 152 (8) , 149.0 (8), 135 (17), 125.0 (32), 111.0 (25), 98.0 (58), 82.0 (45), 71.0(100), 58.0 (97), 55.0 (99).

### B. Am Ringperimeter substituierte monocyclische Systeme

**1.8. 4-Methylcyclotetradecanon und 2-Methylcyclotetradecanon** aus (*syn*/*anti*)-2-Methyl-1-vinyl-1-cyclododecanol 0.8 g (4.4 mmol) 2-Methyl-1-vinyl-1-cyclododecanol, Quartzreaktor 25/400 mm, Temperatur 670°C ± 10, Verdampfung bei 120 - 135°C (Luftbadtemperatur) in ca. 15 min. Stickstoffstrom ca. 1.5 - 2 1/h, Vakuum 3 - 5 mbar. Es wurden 0.7 g (87 % Rohausbeute) eines wasserklaren, nahezu farblosen Kondensates erhalten, das gemäss GC- und GC/MS-Analysen als Hauptkomponente zu ca. 60 % 4-Methylcyclotetradecanon sowie 5 % 2-Methylcyclotetradecanon enthält (*m*/*z* jeweils 224), neben 2 - 8 % Cyclododecanon und weiteren Dehydrierungs- und Fragmentierungsprodukten. Gleichartige Produkte wurden auch mit einem Quartzreaktor von 1 m Länge und 25 mm Innendurchmesser bei 570°C ± 10 erhalten.

¹H-NMR (300 MHz, CDCl₃) Hauptkomponente **A**: 2.61 - 2.24 (*m*, 4 H), 1.82 - 0.95 (*m*, 21 H) ; 0.88 (*d*, ³*J* = 6.3 Hz, 3 H). ¹³C-NMR (75 MHz, CDCl₃) : 211.9 (*s*) , 40.9, 38.9, 32.2, 29.7 (4 *t*), 29.5 (*d*), 25.7, 25.6, 25.5, 25.2, 24.9, 24.5, 23.1 (8 *t*), 19.9 (*q*).

Im ¹H-NMR-Spektrum des Gemisches kann die Nebenkomponente **B** (2-Methylcyclotetradecanon, Verh. ca. 1:12) durch ein Dublett bei 1.06 ppm (*J* = 6.7 Hz), sowie im ¹³C-NMR-Spektrum durch eine Carbonylbande bei 215 ppm sowie einem Dublett bei 45.4 ppm und dem Quartett bei 17.0 ppm charakterisiert werden (vgl. II.2.5).

### 1.9. 3-Methylcycloheptadecanon und 5-Methylcycloheptadecanon aus (syn/anti)-3-Methyl-1-vinyl-1-cyclopentadecanol.

0.5 g (2.1 mmol) 3-Methyl-1-vinyl-1-cyclopentadecanol Quartzreaktor 25/400 mm, Temperatur 670°C ± 10, Verdampfung bei 145 - 170°C (Luftbadtemperatur) in ca. 15 min. Stickstoffstrom ca. 2 - 2.5 1/h, Vakuum 5 - 8 mbar. Es wurden ca. 0.4 g eines wasserklaren, nahezu farblosen Kondensates erhalten, das gemäss GC- und GC/MS-Analysen die zwei Hauptkomponenten (ca. 34% und 28 %) 3-Methylcycloheptadecanon sowie 5-Methylcycloheptadecanon, beide mit *m*/*z* 266) enthält, neben geringeren Anteilen an Dehydrierungs- und Fragmentierungsprodukten.

¹H-NMR-Spektrum (300 MHz, CDCl₃) des Gemisches: 2.51 - 2.4 (*m*), 1.7 - 1.1 (*m*); 0.92 (*d, J* = 6.7 Hz), 0.86 (*d*, *J* = 6.3 Hz), Integralverhältnis ca. 1:1. ¹³C-NMR (75 MHz, CDCl₃): 211.2, 211.6 (2 *s*); 31.5 29.0 (2 *d*); 20.6, 20.1 (2 *q*).

EI-MS (GC/MS) von Komponente **A:** 266.1 (25, M⁺•), 248.1 (48), 208.1 (12), 149.0 (15) , 125.0 (19) , 109.0 (45) , 97.0 (52), 69.0 (76), 55.0 (100). Von Komponente **B:** 266.1 (30, M⁺•), 251.1 (18), 237.1 (38), 223.0 (15), 208.1 (12), 149.0 (10), 125.0 (45), 111.0 (32), 97.0 (53), 85 (72), 69.0 (78), 55.0 (100). (Vgl. II.2.4)

### 1.10. 4-Ethylcyclotetradecanon aus 2-Ethyl-1-vinyl-1-cyclododecanol:

1 g (4.2 mmol) 2-Ethyl-1-vinyl-1-cyclododecanol, Quartzreaktor 25/1000 mm, Temperatur 570°C ± 10, Verdampfung bei 135 - 155°C (Luftbadtemperatur) in ca. 15 min. Stickstoffstrom ca. 1.5 - 2 1/h, Vakuum 3 - 5 mbar. Es wurden 0.8 g (80 % Rohausbeute) eines wasserklaren, farblosen Kondensates erhalten, das gemäss GC- und GC/MS-Analysen als Hauptkomponente zu über 60 % 4-Ethylcyclotetradecanon sowie 5 % 2-Ethylcyclotetradecanon enthält (*m*/*z* jeweils 238), neben weiteren Dehydrierungs- und Fragmentierungsprodukten.

¹H-NMR (300 MHz, CDCl₃) 2.65 - 2.2 (*m*, 4 H), 1.85 - 1.10 (*m*, 23 H) ; 0.88 (*t*, ³*J* = 7 Hz, 3 H). ¹³C-NMR (75 MHz, CDCl₃) : 212.2 (*s*); 41.5, 38.3 (2 *t*); 36.5 *(d);* 29.4,26.3, 26.1, 25.7 (2), 25.6 (2) , 25.0, 24.7, 23.6, 22.6 (11 *t*), 11.0 (*q*)*.* EI-MS (GC/MS) : 238.1 (*M⁺*•).

### 2.1. (R/S)-3-Methylcyclotetradecanon (Normuskon) aus (E/Z)-1-(1-Propenyl)cyclododecanol.

2.3 g (10.2 mmol) (*E*/*Z*)-1-(1-Propenyl)cyclododecanol Quartzreaktor 25/400 mm, Temperatur 660°C ± 10, Verdampfung bei 120 - 135°C (Luftbadtemperatur) in ca. 15 min. Stickstoffstrom ca. 0.5 - 1 1/h, Vakuum 3 - 5 mbar. Es wurden 2.0 g (87 % Rohausbeute) eines wasserklaren, nahezu farblosen Kondensates erhalten, das gemäss GC- und GC/MS-Analysen als Hauptkomponente zu ca. 45 - 55 % 3-Methylcyclotetradecanon enthält, neben 2 - 8 % Cyclododecanon und 4 - 10 % Ausgangsmaterial sowie in kleineren Anteilen Dehydrierungs- und Fragmentierungsprodukte. Durch chromatographische Trennung des Gemisches an Kieselgel (Hexan/TBME 97:3) wurden 0.77 g 3-Methylcyclotetradecanon (33 % isol. Ausb.) als farbloses, beim Lagern im Kühlschrank bei 4°C allmählich kristallisierendes Öl mit typisch moschusartigem Geruch erhalten.

¹H-NMR (300 MHz, CDCl₃) : 2.45 - 2.36 (*m*, 3 H; Feinanalyse zeigt 2.43 (*dd*, ²*J* = 15 Hz, ³*J* = 5 Hz, 1 H, *H*ₐ-C(2)) , 2.41 (*t*, ³*J* = 6.7 Hz, 2 H, *H*₂-C(14) ) ; 2.19 *(dd,* ²*J* = 15 Hz, ³*J* = 5 Hz, 1 H, *H*_{b}-C(2); 2.10 (*m*_{c}, 1 H, *H*-C (3)), 1.64 (*m*_{c}, 2 H, *H*₂C(13)), 1.37 - 1.20 (br. *m*, 18 H); 0.93 (*d*, ³*J* = 6.7 Hz, 3 H, *H*₃C-C(2)). ¹³C-NMR (75 MHz, CDCl₃) : 212.0 (*s*), 49.5, 41.0, 33.7 (3 *t*), 28.8 (*d*), 26.3, 26.1, 25.6, 25.4, 25.3, 25.1, 24.7, 23.4, 22.3 (9 *t*), 20.7 (*q*). MS (GC/MS) : 226.2 (2, M+2), 225.1 (13, *M*+1), 224.1 (50, M⁺. ), 209.1 (28), 195.1 (30), 181.0 (15), 166.0 (38), 125.0 (52), 111.0 (48), 97.0 (50), 85 (100), 71 (52), 55 (51).

### 2.2. (R/S)-3-Methylcyclopentadecanon (Muskon) aus (E/Z)-1-(1-Propenyl)cyclotridecanol.

2.4 g (10 mmol) (*E*/*Z*)-1-(1-Propenyl)cyclotridecanol, Quartzreaktor 25/400 mm, Temperatur 660°C ± 10, Verdampfung bei 140 - 155°C (Luftbadtemperatur) in ca. 15 min. Stickstoffstrom ca. 1.5 - 2.5 1/h, Vakuum 4 - 6 mbar. Es wurden 1.9 g (79 % Rohausbeute) eines wasserklaren, nahezu farblosen Kondensates erhalten, das gemäss GC- und GC/MS-Analysen als Hauptkomponente zu ca. 45 - 55 % 3-Methylcyclopentadecanon enthält, neben 2 - 6 % Cyclododecanon und 4 - 10 % Ausgangsmaterial sowie in kleineren Anteilen Dehydrierungs- sowie Fragmentierungsprodukte.

Durch chromatographische Trennung des Gemisches an Kieselgel (Hexan/TBME 97:3) wurden 0.68 g 3-Methylcyclopentadecanon (28 % isol. Ausb.) als farbloses Öl mit charakteristisch moschusartigem Geruch erhalten, das identische Retentionszeiten und spektroskopische Eigenschaften wie eine Referenzprobe von rac. Muskon aufweist.

¹H-NMR (300 MHz, CDCl₃) : 2.46 - 2.38 (*m*, 3 H; Feinanalyse zeigt 2.43 (*d*, ²*J* = 15 Hz, *H*ₐ-C(2)), 2.41 (*t*, ³*J* = 6.7 Hz, 2 H, H₂-C(15)) ; 2.17 (*dd,* ²*J* = 15 Hz, ³*J* = 5.2 Hz, 1 H, *H*_{b}-C (2) ; 2.04 (*m_{c}*, 1 H, *H*-C(3)) , 1.64 (*m*_{c}, 2 H, *H*₂C(14)), 1.36 - 1.22 (br., *s-*artiges *m*, 20 H); 0.94 (*d*, ³*J* = 6.7 Hz, 3 H, *H*₃C-C(2)). ¹³C-NMR (75 MHz, CDCl₃) : 212.0 (*s*), 50.4, 42.1, 35.6 (3 *t*), 29.0 (*d*), 27.6, 27.1, 26.8, 26.7, 26.6, 26.5, 26.3, 26.2, 25.1, 23.0 (10 *t*), 21.0 (*q*). MS (GC/MS) : 240.2 (3), 239.1 (9), 238.1 (21, M⁺•), 223.1 (12), 209.1 (18), 195.1 (5), 180.1 (8), 125.0 (25), 111.0 (18), 97.0 (35), 85 (45), 69 (48), 55 (100).

### 2.3 (R/S)-3-Methylcyclohexadecanon aus (E/Z)-1-(1-Propenyl)cyclotretradecanol.

Isol. Ausbeute 29%.

¹H-NMR (300 MHz, CDCl₃) : 2.44 - 2.36 (*m*, 3 H) ; 2.17 (*dd*, ²*J* = 15 Hz, ³*J* = 5.2 Hz, 1 H), 2.07 (*m*_{c}, 1 H), 1.58 (*m*_{c}, 2 H), 1.30 - 1.22 (*m*, 22 H); 0.93 (*d*, ³*J* = 6.7 Hz, 3 H). ¹³C-NMR (75 MHz, CDCl₃) : 211.5 (*s*), 50.1, 42.0, 35.4 (3 *t*), 29.0 (*d*), 27.4 (2), 26.9, 26.8, 26.5, 26.4, 26.3, 26.2 (2), 25.3, 22.8 (11 *t*), 20.6 (*q*). MS (GC/MS) : 253.1 (33, *M*+1), 252.1 (52, M⁺•), 223.1 (60), 194.0 (30), 149 (35) 135 (52), 125.0 (88), 111.0 (88), 97.0 (30), 69.0 (95), 55.0 (100).

### 2.4 (R/S)-3-Methylcycloheptadecanon aus (E/Z)-1-(1-Propenyl)cyclopentadecanol.

Isol. Ausbeute 27%.

¹H-NMR (300 MHz, CDCl₃) : 2.43 - 2.35 (*m*, 3 H); 2.17 (*dd*, ²*J* = 15 Hz, ³*J* = 5.2 Hz, 1 H), 2.04 (*m_{c}*, 1 H), 1.58 (*m*_{c}, 2 H), 1.30 - 1.22 (*m*, 24 H); 0.94 (*d*, ³*J* = 6.7 Hz, 3 H). ¹³C-NMR (75 MHz, CDCl₃) : 211.5 (*s*), 50.3, 42.4, 35.8 (3 *t*), 29.0 (*d*), 28.0 (2), 27.6, 27.5, 27.3, 27.1, 26.9, 26.8 (2), 26.7, 25.7, 23.3 (12 *t*), 20.6 (*q*). MS (GC/MS): 267.2 (5, *M*+1), 266.1 (24, M⁺•), 237.1 (12), 136 (13), 125.0 (18), 111.0 (18), 97.0 (30), 85.0 (45), 81.0 (42), 69.0 (53), 55.0 (100).

### 2.5. (R/S)-2-Methylcyclotetradecanon aus 1-(1-Methylethenyl)cyclododecanol.

Isol. Ausbeute 70%.

¹H-NMR (300 MHz, CDCl₃) : 2.70 - 2.34 (*m*, 3 H), 1.81 - 1.15 (*m*, 22 H), 1.05 (*d*, *J* = 7 Hz, 3 H). ¹³C-NMR (75 MHz, CDCl₃): 215.3 (*s*), 45.6 (*d*), 38.4, 33.0, 26.4 (2), 26.1, 25.7, 25.4, 24.9, 24.8, 24.7, 24.6, 21.8 (12 *t*), 17.2 (*q*). EI-MS (GC/MS) : 226.2 (5, *M*+2), 225.1 (35, *M*+1), 224.1 (60, M⁺•), 195.1 (18), 139.0 (30), 111.0 (43), 98.0 (50), 85 (68), 70 (90), 55 (100).

### 2.6. (R/S)-2-Methylcyclotridecanon aus 1-(1-Methylethenyl)-cycloundecanol.

Isol. Ausbeute 60%.

¹H-NMR (300 MHz, CDCl₃) : 2.70 - 2.56 (*m*, 2 H), 2.39 - 2.29 (*m*, 1 H), 1.86 - 1.10 (*m*, 20 H), 1.04 (*d*, *J* = 6.9 Hz, 3 H). ¹³C-NMR (75 MHz, CDCl₃) : 215.4 (*s*), 46.3 (*d*), 40.3, 33.0, 26.6, 26.3, 26.2, 25.6, 25.0, 24.7, 24.4, 24.3, 22.7 (11 *t*), 17.0 (*q*). EI-MS (GC/MS): 212.2 (5, *M*+2), 211.1 (33, *M*+1), 210.1 (45, M⁺•), 181 (33), 153 (35), 139 (32), 111 (34), 97 (35), 72 (38), 55 (100).

### 2.7. 3,3-Dimethylcyclotetradecanon aus 1-(2-Methyl-1-propenyl)cyclododecanol

¹H-NMR (300 MHz, CDCl₃): 2.42 - 2.38 (*t*-artiges *m*, 2 H), 2.34 (*s*, 2 H), 1.64 - 1.59 (*m*, 16 H), 1.55 - 1.23 (*m*, 2 H), 1.14 - 1.09 (*m*, 2 H), 1.00 (*s*, 6 H). ¹³C-NMR (75 MHz, CDCl₃) : 210.4 (*s*), 51.0, 42.3 (2 t), 41.0 (s), 38.8, 33.3 (2 t), 29.5 (2) (q), 27.0, 26.2 (2), 25.6, 25.1, 24.1, 22.6, 22.1 (8 t).EI-MS (GC/MS) : 239.1 (3), 238.0 (8, M⁺•), 223.1 (5), 125.0 (18), 111.0 (22), 97.0 (22), 83 (35), 69 (59), 55 (100).

Als zweite Komponente (29 %) wurde 2-Methyl-2-pentadecen-4-on identifiziert.

### 2.8. (cis/trans)-2,3-Dimethylcyclotetradecanon aus 1-(2-Buten-2-yl)cyclododecanol.

¹H-NMR (300 MHz, CDCl₃) : 2.86 - 2.7 (*m*, 1 H), 2.64 - 2.48 (*m*, 2 H), 2.27 - 1.1 (*m*, 21 H), 1.01 (*d*, J = 6.9 Hz, 3 H), 0.95 (*d*, *J* = 6.9 Hz, 3 H). ¹³C-NMR (75 MHz, CDCl₃) 213.0 (*s*), 51.0 (*d*), 38.1 (*t*), 34.3 (*d*), 29.3, 27.9, 26.2, 25.8, 24.9, 24.8, 24.4, 24.3, 24.1, 20.4 (10 *t*), 17.4, (*q*) 8.8 (*q*) .EI-MS(GC/MS): 238.1 (35, M⁺•), 223.1 (38), 209.1 (80), 191.1 (34), 181.0 (38), 166 (25), 139.0 (100) 125 (80), 111 (85), 98 (88), 83 (95), 69 (92), 55 (98).

### 2.9. 3,4-Dimethylcyclotetradecanon aus (E/Z)-2-Methyl-1-(1-propen-1-yl)-cyclododecanol:

1 g (4.2 mmol) (*E*/*Z*)-2-Methyl-1-(1-propen-1-yl)-cyclododecanol (vgl. I.2.9) enthält ca. 2 - 4 % 2-Methylcyclododecanon), Quartzreaktor 25/1000 mm, Temperatur 570°C ± 10, Verdampfung bei 135 - 155°C (Luftbadtemperatur) in ca. 15 min. Stickstoffstrom ca. 1.5 - 2 1/h, Vakuum 3 - 5 mbar. Es wurden 0.8 g (80 % Rohausbeute) eines wasserklaren, farblosen Kondensates erhalten, das gemäss GC- und GC/MS-Analysen als Hauptkomponenten zu über 50 % isomere 3,4-Dimethylcyclotetradecanone (keine Basislinientrennung) sowie ca. 5 % 2-Methylcyclotetradecanon enthält (*m*/*z* jeweils 238), neben weiteren Dehydrierungs- und Fragmentierungsprodukten.

Diastereomerengemisch (cis/trans-Verbindungen), charakteristische Peaks: ¹H-NMR (300 MHz, CDCl₃) : 0.84 - 0.83 (3 *d*, *J* = 6.5 - 7 Hz), 0.78 (*d*, *J* = 6.6 Hz) .¹³C-NMR (75 MHz, CDCl₃) 211.6/211.3(*s*); 49.3, 44.9, 41.8, 39.8 (4 *t*); 34.0, 33.3, 32.6, 31.2 (4 *q*) ; 32.8 (*t*) ; 28.6 - 24.4 CH₂-Signale (*t*) nicht vollständig aufgelöst; 16.7, 16.1, 14.8, 13.4 (4 *q*).EI-MS (GC/MS): Produktpeaks nicht volltständig getrennt, 238.1/238.1 (M⁺•).

### B. Bicyclische Systeme:

### 3.1. (cis/trans)-Bicyclo[10.4.0]hexadecadecan-2-on aus 1-(1-Cyclohexenyl)cyclododecan-1-ol.

0.5 g 1-(1-Cyclohexenyl)cyclododecan-1-ol (1.9 mmol) wurden bei 140 - 170°C zügig verdampft (Reaktortemperatur 690 - 700°C). Es werden 0.45 g eines leicht gelbfarbenen Öles als Kondensat erhalten, das gemäss GC- und GC/MS-Analysen neben anderen drei neue massenisomere Hauptkomponenten mit *m*/*z* 264 aufweist (36, 9, und 12 %). Eine Trennung der Komponenten durch Chromatographie an Kieselgel (Hexan/TBME 98:2) gelang nur teilweise und es wurden Fraktionen unterschiedlicher Zusammensetzung erhalten (teilweise Kristallisation).

Die isolierte kristalline Fraktion zeigt im GC zwei Signale im Verhältnis von ca. 9:1 (trans/cis-Isomere).

¹H-NMR (300 MHz, CDCl₃) : 2.82 - 2.70 (*m*, 1H), 2.63 - 2.56 (*m*, 1 H), 2.25 - 2.12 (*m*, 1 H), 2.08 - 1.48 (*m*, 7 H), 1.47 - 1.0 (*m*, 22 H). ¹³C-NMR (75 MHz, CDCl₃) Isomer A (Hauptisomer) : 213.2 (*s*), 52.6 (*d*), 37.9 (*t*), 37.0 (*d*), 28.4, 26.2, 26.0 25.9, 25.5, 25.0, 24.5 (2), 24.4, 24.1, 24.0, 23.5, 21.5, 20.7 (14 *t*). Isomer B (schwach): 215.1 (*s*), 57.3 (*d*), 38.7(d).EI-MS(GC/MS): 265.1 (35, M+1), 264.1 (58, M⁺•), 246.1 (10), 209 (15), 137 (18), 125 (40), 96 (48), 81 (52), 67 (55), 55 (100).

Das ebenfalls angefallene ölige Produkt wurde als Cyclohexen-1-yl-undecylketon (ca. 20 %, m/z 264) identifiziert.

### C. Ungesättigte Systeme:

### 4.1. Cyclotetradec-2-enon aus 1-Ethinyl-cyclododecanol.

2.1 g 1-Ethinyl-cyclododecanol (9 mmol) wurden bei 120 - 135 °C innerhalb von ca. 15 min verdampft (Reaktortemperatur 660 - 670 °C). Es wurden 1.8 g Kondensat isoliert, das gemäss GC/MS-Analysen neben ca. 10 % Edukt und ca. 20 % Cyclododecanon mehrere Komponenten mit *m*/*z* 208 als Molekülion-Signal aufweist. Durch Säulenchromatographie an Kieselgel (Hexan/TBME 98:2) wurde die UV-aktive Hauptfraktion (0.6 g, 29 %) als farbloses Öl isoliert:

¹H-NMR (300 MHz, CDCl₃) : 6.83 (*td*, *J* = 15.8, 7.4 Hz, 1 H); 6.20 (*dt J* = 15.8, 1.3 Hz, 1 H) ; 2.53 - 2.48 (*m*, 2 H) ; 2.30 - 2.26 (*m*, 2 H) ; 1.80 - 1.67 (*m*, 2 H), 1.58 - 1.52 (*m*, 2H), 1.45 - 1.2 (*m*, 14 H). ¹³C-NMR (75 MHz, CDCl₃) : 202.1 (*s*), 148.2, 130.3 (2 *d*), 40.4, 31.4, 26.6, 26.3, 26.2, 26.0, 25.8, 25.7, 25.4, 25.0, 24.9 (11 *t*).EI-MS(GC/MS): 209.1 (5, M+1), 208.1 (15, M⁺•), 165 (10), 98 (40), 95 (50), 81 (90), 67 (65), 55 (100).

### 4.2. Cyclopentadec-2-enon aus 1-Ethinyl-cyclotridecanol.

2.0 g 1-Ethinyl-cyclotridecanol (9 mmol) wurden bei 125 - 130 °C innerhalb von ca. 15 min verdampft (Reaktortemperatur 670 - 690 °C). Es wurden 1.8 g Kondensat isoliert, das gemäss GC/MS-Analysen neben ca. 10 % Edukt und ca. 15 % Cyclotridecanon mehrere Komponenten mit *m*/*z* 222 als Molekülion-Signal aufweist. Durch Säulenchromatographie an Kieselgel (Hexan/TBME 98:2) wurde die UV-aktive Hauptfraktion 0.44 g, (22 %) isoliert:

¹H-NMR (300 MHz, CDCl₃) : 6.81 (*td*, *J* = 15.7, 7.5 Hz, 1 H); 6.19 (*dt J* = 15.7, 1.3 Hz, 1 H); 2.52 - 2.47 (*m*, 2 H); 2.30 - 2.23 (*m*, 2 H) ; 1.72 - 1.44 (*m*, 4H), 1.4 - 1.2 (*m*, 16 H). ¹³C-NMR (75 MHz, CDCl₃) : 201.7 (*s*), 147.9, 130.7 (2 *d*), 40.0, 31.6, 26.9, 26.8, 26.7, 26.6 (2), 26.5, 26.2, 26.0, 25.4, 25.2 (12 *t*). EI-MS (GC/MS): 223.1 (5, M+1), 222.1 (15, M⁺•), 164 (10), 109 (50), 96 (52), 95 (50), 81 (80), 68 (55), 55 (100).

### 4.3. (E/Z)-3-Methylcyclopentadec-2-en-1-on aus 1-(1-Propinyl)cyclotridecanol.

0.8 g 1-(1-Propinyl)cyclododecanol (3.3 mmol) wurden bei 130 - 140 °C verdampft (Reaktortemperatur 660 - 670°C), wobei 0.5 g Kondensat als gelbliches Öl erhalten wurden. GC und GC-MS-Analysen zeigen ein komplexes Produktgemisch, das neben ca. 12 % Cyclotridecanon (m/z 196), ca. 15 - 20% Alkenfraktionen (m/z 218) unter anderem drei Fraktionen (ca. 18, 15, 11%) mit massenisomeren Molekülionenpeaks (m/z 236) enthält. Durch Chromatographie an Kieselgel (Hexan/TBME 98:2) konnte nach Abtrennen der Alkenfraktion ein Teil der zuerst eluierten Ketonfraktion abgetrennt werden (farbloses Öl) :

¹H-NMR (300 MHz, CDCl₃) : 6.09 (br. *s*, 1H), 2.76 (*t*, *J* = 6.8 Hz, 2 H), 2.46 - 2.35 (*m*, 2 H), 1.86 (*d*, *J* = 1.3 Hz, 3 H), 1.74 - 1.2 (*m*, 20 H). ¹H-NMR (75 MHz, CDCl₃) : 201.8 (*s*), 158.4 (*s*), 125.0 (*d*), 41.8 (*t*), 31.6, 29.3, 27.0, 26.9, 26.8, 26.7, 26.5, 26.3, 26.1, 25.3, (10 *t*) 25.1 (*q*), 23.8 (*t*). EI-MS (GC/MS) : 237.1 (5, M+1), 236.0 (18, M⁺•), 221.0 (12), 123.0 (13), 109 (46), 98 (87), 95 (100), 83 (90), 67 (52), 55 (80).

Die nachfolgenden, durch Säulenchromatogrophie an Kieselgel nicht ausreichend aufgetrennten Produktfraktionen wurden vereinigt. Nach katalytischer Hydrierung dieses Gemisches mit Palladium (10%) auf Aktivkohle in Essigsäureethylester und anschliessender erneuter Säulenchromatographie an Kieselgel (Hexan/TBME 98 : 2) wurden drei Hauptfraktionen erhalten, von denen die eine (0.15 g, 19 % ) sich duch GC-, GC/MS-Analysen und NMR-Spektroskopie als identisch mit 3-Methyl-cyclopentadecanon (Muskon) erwies (vgl. II.2.2.). Die weiteren Produke (Anteil ca. 10 bzw. 15 %) wurden als Cyclotridecanon bzw. Hexadecan-4-on identifiziert.

### 4.4. (E/Z)-3-Methylcyclotetradec-2-en-1-on aus 1-(1-Propinyl)cyclododecanol.

1.1 g 1-(1-Propinyl)cyclododecanol (5 mmol) wurden bei 130 - 140 °C verdampft (Reaktortemperatur 660 - 680°C), wobei 0.9 g Kondensat als gelbliches Öl erhalten wurden. GC und GC-MS-Analysen zeigen ein komplexes Produktgemisch, das neben ca. 15% Cyclododecanon (*m*/*z* 182), ca. 15 - 20% Alkenfraktionen (*m*/*z* 204) unter anderem drei Fraktionen (ca. 25, 20, 15%) mit massenisomeren Molekülionenpeaks (m/z 222) enthält.

¹H-NMR-Spektrum dieses Gemisches: 6.21 (*s*), 6.09 (*s*), 2.78 (*t*, *J* = 6. 8Hz), 2.6 - 2.35 (*m*), 2.13 (*d*, *J* = 1 Hz), 2.02 (*s*), 1.95 - 1.15 (*m*), 0.88 (*t, J* = 6.8 Hz).

Nach katalytischer Hydrierung dieses Gemisches mit Palladium (10%) auf Aktivkohle in Essigsäureethylester und anschliessender Säulenchromatographie an Kieselgel (Hexan/TBME 98 : 2) wurden zwei Hauptfraktionen erhalten, von denen die eine (0.35 g, 32 %) sich duch GC-, GC/MS-Analysen und NMR-Spektroskopie als identisch mit 3-Methyl-cyclotetradecanon erwies. Das zweite Produkt (Anteil ca. 15 %) wurde als Pentadecan-4-on identifiziert.

### 5.1. (R/S)-3-Methyl-cyclotetradecanon aus (E/Z)-1-(Trimethylsilyloxy)-1-(1-propenyl)-cyclododecanol und anschliessende Hydrodrolyse der cyclischen Trimethylsilylenolether-Zwischenstufe:

1.5 g *(E*/*Z*)-1-Propen-1-yl-1-Trimethylsilyloxycoclododecan (5 mmol, *E*:*Z*-Isomerenverhältnis gemäss ¹H-NMR und GC ca. 3:2) wurden bei 120 - 130 °C innerhalb 15 min verdampft (Reaktortemperatur 660 - 670°C). 1.3 g Kondensat (86 %) als farbloses Öl. Gemäss GC/MS-Analysen Umsetzung ca. 90 %, wobei neben insgesamt ca. 30 - 40 % Alkenfraktionen (Desilylierung, M⁺ • = 206) mit ca. 25 bzw. 15 % jeweils zwei neue verbreiterte Produkt-Signalpaare mit m/z 296 (M⁺•, Trimethylsilylenolether-Isomere) beobachtet wurden

EI-MS: 297.2 (5), 296.2 (15, M⁺•), 281.2 (20), 253.1 (10), 197.0 (18), 169.0 (60), 73.0 (100).

Das erhaltene Rohkondensat wurde in 20 ml THF gelöst und unter Rühren mit einigen Tropfen verd. Schwefelsäure und einer verd. wässr. Kaliumfluorid-Lösung versetzt. Nach Rühren über Nacht wurde auf Wasser gegossen, mit Hexan extrahiert, und nach üblicher Aufarbeitung an Kieselgel chromatographiert (Hexan/TBME 97:3). Neben einer UV-aktiven Vorfraktion (Alkenfraktion) werden 0.42 g (28 %) eines farblosen Öles erhalten, das sich duch GC-, GC/MS-Analysen und NMR-Spektroskopie als identisch mit dem bereits zuvor hergestellten 3-Methylcyclotetradecanon erwies

## Patentansprüche

1. Thermo-Isomerisationsverfahren zur Herstellung von makrocyclischen Ketonen der Formel Ia, oder Ib 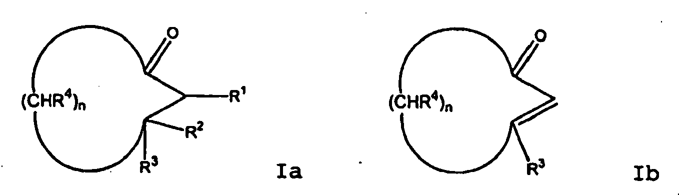 wobei
R¹, R², R³ unabhängig voneinander Wasserstoff oder eine C₁ bis C₆-Alkyl-Gruppe darstellen,
R¹ und R² oder R² und R³ in Formel Ia unabhängig voneinander einen Ring bilden können,
R⁴ Wasserstoff, eine lineare oder verzweigte C₁ bis C₄-AlkylGruppe darstellt,
n eine ganze Zahl ist, und
n 7 bis 14 ist,
durch
(a) Überführung eines makrocyclischen tertiären Allylalkohols der Formel IIa oder Propargylalkohols der Formel IIb 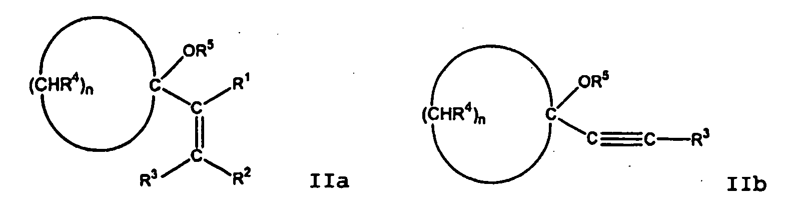 wobei
R¹, R², R³, R⁴, und n die gleiche Bedeutung wie oben haben,
R⁵ entweder Wasserstoff oder eine Trialkylsilyl-Gruppe oder ein Alkalimetallkation darstellt,
bei 100-300°C in die Gasphase, und
(b) Erhitzen des in die Gasphase überführten makrocyclischen tertiären Allylalkohols der Formel IIa oder Propargylalkohols der Formel IIb auf Temperaturen von 500 bis 700°C, und
(c) falls R⁵ eine Trialkylsilyl-Gruppe ist, Hydrolyse des unter (b) entstandenen Trialkylsilylethers in das entsprechende Keton der Formel Ia oder Ib.

2. Thermo-Isomerisationsverfahren gemäss Anspruch 1, wobei R¹, R² und R³ je für Methyl-Gruppen stehen.

3. Thermo-Isomerisationsverfahren gemäss einem der vorangehenden Ansprüchen **dadurch gekennzeichnet, dass** der makrocyclische tertiäre Allylalkohol der Formel IIa oder Propargylalkohol der Formel IIb vor dem Überführen in die Gasphase in einem inerten Lösungsmittel gelöst wird.

4. Thermo-Isomerisationsverfahren gemäss einem der vorangehenden Ansprüchen **dadurch gekennzeichnet, dass** der makrocyclische tertiäre Allylalkohol der Formel IIa oder Propargylalkohol der Formel IIb kontinuierlich in die Gasphase übergeführt wird.

5. Thermo-Isomerisationsverfahren gemäss einem der vorangehenden Ansprüchen **dadurch gekennzeichnet, dass** der makrocyclische tertiäre Allylalkohol der Formel IIa oder Propargylalkohol der Formel IIb bei 120-250°C in die Gasphase übergeführt wird.

6. Thermo-Isomerisationsverfahren gemäss einem der vorangehenden Ansprüchen **dadurch gekennzeichnet, dass** zu dem makrocyclischen tertiären Allylalkohol der Formel IIa oder Propargylalkohol der Formel IIb in der Gasphase ein Inertgas zugegeben wird.

7. Thermo-Isomerisationsverfahren gemäss einem der vorangehenden Ansprüchen **dadurch gekennzeichnet, dass** der makrocyclische tertiäre Allylalkohol der Formel IIa oder Propargylalkohol der Formel IIb in der Gasphase auf 500 bis 670°C erhitzt wird.

8. Makrocyclische Ketone der Formel Ia ausgewählt aus der Gruppe von
2-Methylcyclotetradecanon
3-Methylcycloheptadecanon
5-Methylcycloheptadecanon
3-Methylcycloheptadecanon
4-Ethylcyclotetradecanon
3,4-Dimethylcyclotetradecanon.

9. Makrocyclischer tertärer Allylalkohol der Formel IIa
1-Vinyl-1-cycloundecanol
1-Vinyl-i-cyclotridecanol
1-Vinyl-1-cyclotetradecanol
1-Vinyl-1-cyclopentadecanol
(syn/anti)-2-Methyl-1-vinyl-1-cyclododecanol
(syn/anti)-2-Ethyl-1-vinyl-1-cyclododecanol
(syn/anti)-3-Methyl-1-vinyl-1-cyclopentadecanol
(E/Z)-1-(1-Propen-1-yl)cyclododecanol
(E/Z)-1-(1-Propenyl)cyclotridecanol
(E/Z)-1-(1-Propenyl)cyclotetradecanol
(E/Z)-1-(1-Propen-1-yl)cyclopentadecanol
1-(1-Methylethenyl)cycloundecanol
1-(2-Methyl-1-popenyl)cyclododecanol
(E/Z)-1-(2-Buten-2-yl)cyclododecanol
(E/Z)-1-(Trimehylsilyloxy)-1-(1-propenyl)cyclododecanol)
(E/Z)-2-Methyl-1-(1-propen-1-yl)cyclododecanol.

10. Makrocyclische tertiäre Propargylalkohole der Formel IIb
1-Ethinylcyclotridecanol
1-(1-Propinyl)cyclododecanol

## Claims

1. Thermo-isomerization method for producing macrocyclic ketones of the formula Ia, or Ib 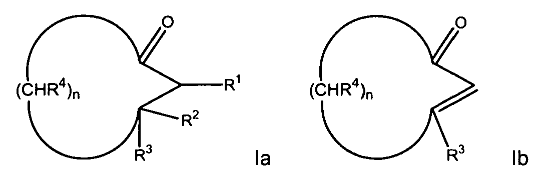 wherein
R¹, R², and R³ are independently hydrogen, or a C₁ to C₆ alkyl group,
R¹ and R² or R² and R³ in formula Ia, independently of one another, can form a ring,
R⁴ is hydrogen, a linear or branched C₁ to C₄ alkyl group,
n is an integer of 7 to 14,
by:
(a) converting a macrocyclic tertiary allyl alcohol of the formula IIa or propargyl alcohol of the formula IIb 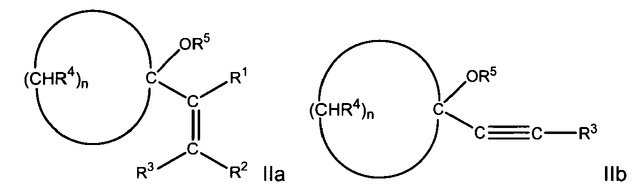 wherein
R¹, R², R³, R⁴, and n have the same meanings as above,
R⁵ is either hydrogen or a trialkylsilyl group or an alkali metal cation,
at 100 - 300°C into the gas phase, and
(b) heating the macrocyclic tertiary allyl alcohol of the formula IIa or propargyl alcohol of the formula IIb, converted into the gas phase, to temperatures of from 500 to 700°C, and
(c) if R⁵ is a trialkylsilyl group, hydrolysis of the trialkylsilyl ether resulting under (b) into the corresponding ketone of the formula la or Ib.

2. Thermo-isomerization method according to claim 1, wherein R¹, R² and R³ are methyl.

3. Thermo-isomerization method according to any of the preceding claims, **characterized in that** the macrocyclic tertiary allyl alcohol of formula IIa or propargyl alcohol of formula IIb is dissolved in an inert solvent prior to be converted into the gas phase.

4. Thermo-isomerization method according to any of the preceding claims, **characterized in that** the macrocyclic tertiary allyl alcohol of formula IIa or propargyl alcohol of formula IIb is converted into the gas phase continuously.

5. Thermo-isomerization method according to any of the preceding claims, **characterized in that** the macrocyclic tertiary allyl alcohol of formula IIa or propargyl alcohol of formula IIb is converted into the gas phase at 120-250°C.

6. Thermo-isomerization method according to any of the preceding claims, **characterized in that** an inert gas is added to the macrocyclic tertiary allyl alcohol of formula IIa or propargyl alcohol of formula IIb in the gas phase.

7. Thermo-isomerization method according to any of the preceding claims, **characterized in that** the macrocyclic tertiary allyl alcohol of formula IIa or propargyl alcohol of formula IIb is heated to 500 to 670°C in the gas phase.

8. Macrocyclic ketones of formula la selected from the group consisting of 2-methylcyclotetradecanone, 3-methylcycloheptadecanone, 5-methylcycloheptadecanone, 3-methylcycloheptadecanone, 4-ethylcyclotetradecanone, and 3,4-dimethylcyclotetradecanone.

9. Macrocyclic tertiary allyl alcohol of formula IIa selected from 1-vinyl-1-cycloundecanol, 1-vinyl-1-cyclotridecanol, 1-vinyl-1-cyclotetradecanol, 1-vinyl-1-cyclopentadecanol, (syn/anti)-2-methyl-1-vinyl-1-cyclododecanol, (syn/anti)-2-ethyl-1-vinyl-1-cyclododecanol, (syn/anti)-3-methyl-1-vinyl-1-cyclopentadecanol, (E/Z)-1-(1-propen-1-yl)cyclododecanol, (E/Z)-1-(1-propenyl)cyclo tridecanol, (E/Z)-1-(1-propenyl)cyclotetradecanol, (E/Z)-1-(1-propen-1-yl)cyclopentadecanol, 1-(1-methylethenyl) cycloundecanol, 1-(2-methyl-1-propenyl)cyclododecanol, (E/Z)-1-(2-buten-2-yl)cyclododecanol, (E/Z)-1-(trimethylsilyloxy)-1-(1-propenyl)cyclododecanol), and (E/Z)-2-methyl-1-(1-propen-1-yl)cyclododecanol.

10. Macrocyclic tertiary propargyl alcohols of formula IIb selected from 1-ethynylcyclotridecanol, and 1-(1-propynyl)cyclododecanol.

## Revendications

1. Procédé de thermo-isomérisation pour la préparation de cétones macrocycliques de formule Ia, ou Ib 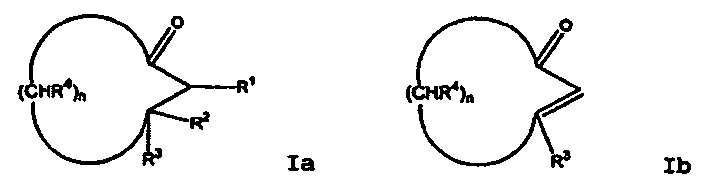 où
R¹, R² R³ représentent, indépendamment l'un de l'autre, hydrogène ou un groupe C₁-C₆-alkyle,
R¹ et R² ou R² et R³ dans la formule Ia peuvent former, indépendamment l'un de l'autre, un cycle,
R⁴ représente hydrogène, un groupe C₁-C₄-alkyle linéaire ou ramifié,
n vaut un nombre entier, et
n vaut 7 à 14,
par
(a) transfert d'un alcool allylique tertiaire macrocyclique de formule IIa ou d'un alcool propargylique tertiaire macrocyclique de formule IIb 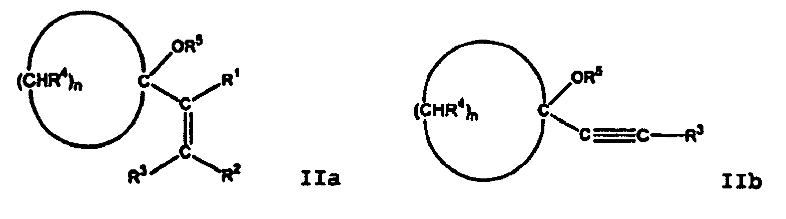 où
R¹, R², R³ R⁴et n , ont la même signification que ci-dessus,
R⁵ représente hydrogène ou un groupe trialkylsilyle ou un cation de métal alcalin,
à 100-300°C dans la phase gazeuse, et
(b) chauffage de l'alcool allylique tertiaire macrocyclique de formule IIa ou de l'alcool propargylique tertiaire macrocyclique de formule IIb transféré dans la phase gazeuse à des températures de 500 à 700°C, et
(c) si R⁵ représente un groupe trialkylsilyle, hydrolyse du trialkylsilyléther formé au point (b) en cétone correspondante de formule Ia ou Ib.

2. Procédé de thermo-isomérisation selon la revendication 1, où R¹, R² et R³ représentent chacun un groupe méthyle.

3. Procédé de thermo-isomérisation selon l'une quelconque des revendications précédentes, **caractérisé en ce que** l'alcool allylique tertiaire macrocyclique de formule IIa ou l'alcool propargylique tertiaire macrocyclique de formule IIb est dissous dans un solvant inerte avant le transfert dans la phase gazeuse.

4. Procédé de thermo-isomérisation selon l'une quelconque des revendications précédentes, **caractérisé en ce que** l'alcool allylique tertiaire macrocyclique de formule IIa ou l'alcool propargylique tertiaire macrocyclique de formule IIb est transféré en continu dans la phase gazeuse.

5. Procédé de thermo-isomérisation selon l'une quelconque des revendications précédentes, **caractérisé en ce que** l'alcool allylique tertiaire macrocyclique de formule IIa ou l'alcool propargylique tertiaire macrocyclique de formule IIb est transféré dans la phase gazeuse à 120-250°C.

6. Procédé de thermo-isomérisation selon l'une quelconque des revendications précédentes, **caractérisé en ce qu'**on ajoute un gaz inerte à l'alcool allylique tertiaire macrocyclique de formule IIa ou à l'alcool propargylique tertiaire macrocyclique de formule IIb dans la phase gazeuse.

7. Procédé de thermo-isomérisation selon l'une quelconque des revendications précédentes, **caractérisé en ce que** l'alcool allylique tertiaire macrocyclique de formule IIa ou l'alcool propargylique tertiaire macrocyclique de formule IIb est chauffé dans la phase gazeuse à 500-670°C.

8. Cétones macrocycliques de formule Ia choisies dans le groupe formé par
la 2-méthylcyclotétradécanone
la 3-méthylcycloheptadécanone
la 5-méthylcycloheptadécanone
la 3-méthylcycloheptadécanone
la 4-éthylcyclotétradécanone
la 3,4-diméthylcyclotétradécanone.

9. Alcool allylique tertiaire macrocyclique de formule IIa
1-vinyl-1-cyclo-undécanol
1-vinyl-1-cyclotridécanol
1-vinyl-1-cyclotétradécanol
1-vinyl-1-cyclopentadécanol
(syn/anti)-2-méthyl-1-vinyl-1-cyclododécanol
(syn/anti)-2-éthyl-1-vinyl-1-cyclododécanol
(syn/anti)-3-méthyl-1-vinyl-1-cyclopentadécanol
(E/Z)-1-(1-propén-1-yl)cyclododécanol
(E/Z)-1-(1-propényl)cyclotridécanol
(E/Z)-1-(1-propényl)cyclotétradécanol
(E/Z)-1-(1-propén-1-yl)cyclopentadécanol
1-(1-méthyléthényl)cyclo-undécanol
1-(2-méthyl-1-propényl)cyclododécanol
(E/Z)-1-(2-butén-2-yl)cyclododécanol
(E/Z)-1-(triméthylsilyloxy)-1-(1-propényl)cyclododécanol)
(E/Z)-2-méthyl-1-(1-propén-1-yl)cyclododécanol.

10. Alcools propargyliques tertiaires macrocycliques de formule IIb
1-éthynylcyclotridécanol
1-(1-propynyl)cyclododécanol.
